# EUROPEAN PATENT APPLICATION

(11) **EP 4 548 976 A1**
(43) Date of publication of application: **07.05.2025**
(21) Application number: 23383104.9
(22) Date of filing: 30.10.2023
(51) Int. Cl.: A61P 31/12, C07D 209/08, C07D 401/12, C07D 403/12, C07D 405/12, C07D 405/14, C07D 409/12, C07D 413/12, C07D 471/04, C07D 495/04, A61K 31/404

(54) **ANTIVIRAL COMPOUNDS AND COMPOSITIONS THEREFROM**

(71) Applicant: Consejo Superior De Investigaciones Científicas, 28006 Madrid (ES)
(72) Inventor: GUTIÉRREZ RODRÍGUEZ, Marta, 28006 Madrid (ES); GASTAMINZA LANDART, Pablo, 28049 Madrid (ES); GARAIGORTA DE DIOS, Urtzi, 28049 Madrid (ES); CABRERA TORREJÓN, Daniel, 28006 Madrid (ES); CASTRO NAVAS, Francisco Fermín, 28006 Madrid (ES); BUENO FERNÁNDEZ, Paula, 28049 Madrid (ES)
(74) Representative: ABG Intellectual Property Law, S.L.

(57) **Abstract**

The present invention relates to a compound of formula (I) or a pharmaceutically acceptable salt thereof. It also relates to a compound of formula (I) or a pharmaceutically acceptable salt thereof for use in medicine, in particular for the prevention of treatment of infection by coronavirus. The invention also relates to a process for the preparation of a compound of formula (I) or a pharmaceutically acceptable salt thereof

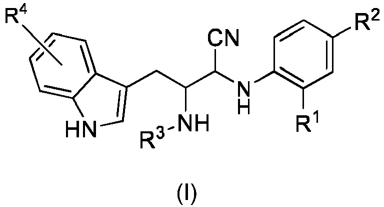

## Description

### FIELD OF THE INVENTION

The present invention relates to 3-amino-4-(1H-indol-3-yl)-2-(phenyl-amino)butanenitrile compounds of formula (I) or pharmaceutically acceptable salts thereof. It also relates to compounds of formula (I) or pharmaceutically acceptable salts thereof for use in medicine, in particular for the prevention or treatment of infection by coronavirus. The invention also relates to a process for the preparation of compounds of formula (I) or pharmaceutically acceptable salts thereof.

### BACKGROUND

Different types of coronavirus affecting human beings are known in the art. These viruses are characterized for having a corona-shaped spike on their surface. Common coronaviruses are 229E (alpha coronavirus), NL63 (alpha coronavirus), OC43 (beta coronavirus) and HKU1 (beta coronavirus). Such viruses commonly infect human beings. Certain coronaviruses affecting human beings come from the evolution of animal coronavirus. Such viruses may provoke more severe conditions, such as respiratory syndromes. Recent examples of such coronaviruses are MERS-CoV (the beta coronavirus that causes Middle East Respiratory Syndrome, or MERS), SARS-CoV (the beta coronavirus that causes severe acute respiratory syndrome, or SARS) and SARS-CoV-2 (the coronavirus that caused coronavirus disease 2019, or COVID-19).

Because most coronaviruses are new, the degree of immunity to such viruses still remain low, so that these coronaviruses have the potential to infect a large number of people. Despite the incidence of severe cases with acute respiratory syndromes is rather low, the large potential for global contamination provides for a potential to generate a large number of cases with severe symptoms. Consequently, there is a need for anti-infective agents allowing for preventing or treating infection with coronaviruses.

Several agents suitable for the treatment or prevention of diseases provoked by coronavirus infection are known in the art. For instance, remdesivir was shown to interfere in RNA polymerization of SARS-coronavirus and MERS-coronavirus in vitro, thereby being suitable for preventing the development of severe forms of coronavirus disease infection. Remdesivir has been approved by the US Food and Drug Administration for the treatment of infection by SARS-Cov-2.

Other anti-infective agents are known in the art. For instance, it has been reported as well that the combination of nirmatrelvir and ritonavir (commercialized with the name paxlovid) prevents the reproduction of the virus by inhibition of an enzyme. Other small molecules with anti-infective properties against coronavirus infection include ivermectin, which is a mixture of avermectins B1a and B1b, molnupiravir and nitazoxadin.

Certain derivatives of 3-amino-4-(1H-indol-3-yl)-2-(phenylamino)butanenitrile are known in the art. For instance, Herrero, S. et al., Journal of Organic Chemistry (2003), 68(11), 4582-4585 reports the compound of formula (I1) as a 2:1 mixture of (S,S) and (S,R) diastereomers

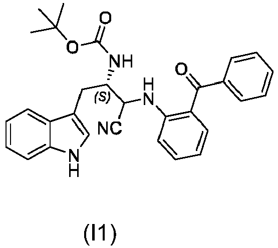

These compounds are disclosed as synthetic intermediates in the synthesis of 5-phenyl-1,4-benzodiazepine derivatives. The authors are however silent about the use of these compounds of formula (I1) in medicine or about any biological activity for said compounds.

Additionally, Herrero S. et al., Tetrahedron (2003), 59(25), 4491-4499 discloses the compound of formula (I2)

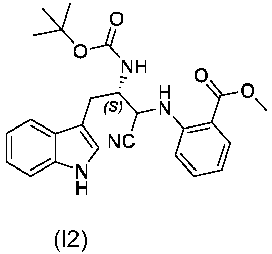

This compound is disclosed as synthetic intermediate in the synthesis of chiral 2-substituted-5-oxo-1,2,3,4-tetrahydro-5H-1,4-benzodiazepines. The authors are however silent about the use of the compound of formula (I2) in medicine or about any biological activity for said compound.

From what is disclosed in the art, it derives that there is still a need for the provision of small molecules with anti-viral activity, in particular for use in the treatment or prevention of infection with coronavirus.

### SUMMARY OF THE INVENTION

After exhaustive research, the inventors have found that certain 2,3-diamino-4-(1*H*-indol-3-yl)butanenitrile derivatives unexpectedly exhibit anti-viral activity and are useful for the treatment or prevention of infection with coronavirus.

Thus, in a first aspect, the invention relates to a compound of formula (I) or a pharmaceutically acceptable salt thereof 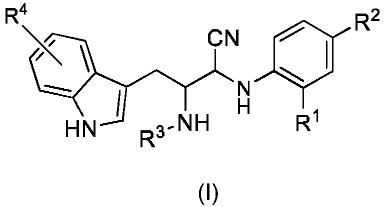
wherein R¹ is a group selected from the group consisting of hydrogen, halogen, carboxyl (-COOH), (C₁-C₄)alkyloxycarbonyl, (C₁-C₄)alkylcarbonyl, phenylcarbonyl and phenyl optionally substituted by a group selected from -CO-benzoimidazole and -CO-(5-7 saturated heterocycle comprising 1 or 2 heteroatoms selected from the group consisting of O, N and S);
R² is a group selected from the group consisting of:
   a) hydrogen,
   b) halogen,
   c) a cycle selected from the group consisting of pyridinyl, pyrimidinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, 2,3-dihydrobenzo[b][1,4]dioxine and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group -G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom;
R³ is a group *tert*-butoxycarbonyl; and
R⁴ is a group selected from the group consisting of hydrogen, halogen, C₁₋₄-alkynyl and 4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl
with the proviso that the compound is not one of the following compounds or their pharmaceutically acceptable salts:
   methyl 4-(((2S)-2-((tert-butoxycarbonyl)amino)-1-cyano-3-(1H-indol-3-yl)propyl)-amino)-4'-morpholino-[1,1'-biphenyl]-3-carboxylate;
   methyl 5-bromo-2-(((2S)-2-((tert-butoxycarbonyl)amino)-1-cyano-3-(1H-indol-3-yl)propyl)amino)benzoate;
   (3S)-2-((4-Bromo-2-benzoylphenyl)amino)-4-(1H-indol-3-yl)-3-(tert-butoxycarbonylamino) butanenitrile;
   (3S)-3-(tert-Butoxycarbonylamino)-4-(1H-indol-3-yl)-2-((2-benzoyl-4-chlorophenyl)amino) butanenitrile;
   methyl 5-bromo-2-(((2S)-2-((tert-butoxycarbonyl)amino)-1-cyano-3-(1H-indol-3-yl)propyl)amino)benzoate;
   methyl 2-(((2S)-2-((tert-butoxycarbonyl)amino)-1-cyano-3-(1H-indol-3-yl)propyl)amino)-5-(2,3-dihydrobenzo[b][1,4]dioxin-6-yl)benzoate;
   methyl 4-(((2S)-2-((tert-butoxycarbonyl)amino)-1-cyano-3-(1H-indol-3-yl)propyl)amino)-3'-morpholino-[1,1'-biphenyl]-3-carboxylate;
   ethyl 2-(((2S)-2-((tert-butoxycarbonyl)amino)-1-cyano-3-(1H-indol-3-yl)propyl)amino)-benzoate;
   methyl 4-bromo-2-(((2S)-2-((tert-butoxycarbonyl)amino)-1-cyano-3-(1H-indol-3-yl)propyl)-amino)benzoate;
   methyl 4-(((2S)-2-((tert-butoxycarbonyl)amino)-1-cyano-3-(1H-indol-3-yl)propyl)amino)-4'-(tert-butyl)-[1,1'-biphenyl]-3-carboxylate;
   methyl 2-bromo-6-(((2S)-2-((tert-butoxycarbonyl)amino)-1-cyano-3-(1H-indol-3-yl)propyl)-amino)benzoate;
   methyl 4-(((2S)-2-((tert-butoxycarbonyl)amino)-1-cyano-3-(1H-indol-3-yl)propyl)amino)-4'-ethyl-[1,1'-biphenyl]-3-carboxylate;
   methyl 4-(((2S)-2-((tert-butoxycarbonyl)amino)-1-cyano-3-(1H-indol-3-yl)propyl)amino)-4'-cyclopropyl-[1,1'-biphenyl]-3-carboxylate;
   methyl 4-(((2S)-2-((tert-butoxycarbonyl)amino)-1-cyano-3-(1H-indol-3-yl)propyl)amino)-4'-(4-methylpiperazin-1-yl)-[1,1'-biphenyl]-3-carboxylate;
   methyl 4-(((2S)-2-((tert-butoxycarbonyl)amino)-1-cyano-3-(1H-indol-3-yl)propyl)amino)-2'-fluoro-5'-isopropoxy-[1,1'-biphenyl]-3-carboxylate;
   methyl 2-(((2S)-2-((tert-butoxycarbonyl)amino)-1-cyano-3-(1H-indol-3-yl)propyl)amino)-5-(6-(pyrrolidin-1-yl)pyridin-3-yl)benzoate;
   methyl 2-(((2S)-2-((tert-butoxycarbonyl)amino)-1-cyano-3-(1H-indol-3-yl)propyl)amino)-5-(6-methylpyridin-3-yl)benzoate;
   methyl 2-(((2S)-2-((tert-butoxycarbonyl)amino)-1-cyano-3-(1H-indol-3-yl)propyl)amino)-5-(6-morpholinopyridin-3-yl)benzoate;
   methyl 2-(((2S)-2-((tert-butoxycarbonyl)amino)-1-cyano-3-(1H-indol-3-yl)propyl)amino)-5-(6-methoxypyridin-3-yl)benzoate;
   methyl 2-(((2S)-2-((tert-butoxycarbonyl)amino)-1-cyano-3-(1H-indol-3-yl)propyl)amino) benzoate;
   tert-Butyl 2-(((2S)-2-((tert-butoxycarbonyl)amino)-1-cyano-3-(1H-indol-3-yl)propyl)amino)-benzoate;
   methyl 4-(((2S)-2-((tert-butoxycarbonyl)amino)-1-cyano-3-(1H-indol-3-yl)propyl)amino)-4'-butyl-[1,1'-biphenyl]-3-carboxylate;
   methyl 4-(((2S)-2-((tert-butoxycarbonyl)amino)-1-cyano-3-(1H-indol-3-yl)propyl)amino)-4'-(diethylamino)-[1,1'-biphenyl]-3-carboxylate;
   methyl 4-(((2S)-2-((tert-butoxycarbonyl)amino)-1-cyano-3-(1H-indol-3-yl)propyl)amino)-4'-(cyclopropyl)-[1,1'-biphenyl]-3-carboxylate;
   methyl 4-(((2S)-2-((tert-butoxycarbonyl)amino)-1-cyano-3-(1H-indol-3-yl)propyl)amino)-4'-propoxy-[1,1'-biphenyl]-3-carboxylate;
   methyl 4-(((2S)-2-((tert-butoxycarbonyl)amino)-1-cyano-3-(1H-indol-3-yl)propyl)amino)-3'-(tert-butyl)-[1,1'-biphenyl]-3-carboxylate;
   methyl 4-(((2S)-2-((tert-butoxycarbonyl)amino)-1-cyano-3-(1H-indol-3-yl)propyl)amino)-3'-ethyl-[1,1'-biphenyl]-3-carboxylate;
   methyl 4-(((2S)-2-((tert-butoxycarbonyl)amino)-1-cyano-3-(1H-indol-3-yl)propyl)amino)-3'-(pyrrolidin-1-yl)-[1,1'-biphenyl]-3-carboxylate;
   methyl 4-(((2S)-2-((tert-butoxycarbonyl)amino)-1-cyano-3-(1H-indol-3-yl)propyl)amino)-3'-(dimethylamino)-[1,1'-biphenyl]-3-carboxylate;
   methyl 4-(((2S)-2-((tert-butoxycarbonyl)amino)-1-cyano-3-(1H-indol-3-yl)propyl)amino)-3'-(trifluoromethoxy)-[1,1'-biphenyl]-3-carboxylate;
   methyl 2-(((2S)-2-((tert-butoxycarbonyl)amino)-1-cyano-3-(1H-indol-3-yl)propyl)amino)-5-(6-(4-isopropylpiperazin-1-yl)pyridin-3-yl)benzoate;
   methyl 2-(((2S)-2-((tert-butoxycarbonyl)amino)-1-cyano-3-(1H-indol-3-yl)propyl)amino)-5-(2-ethoxypyrimidin-5-yl)benzoate;
   methyl 4-(((2S)-2-((tert-butoxycarbonyl)amino)-1-cyano-3-(1H-indol-3-yl)propyl)amino)-4'-butyl-[1,1'-biphenyl]-3-carboxylate;
   methyl 3'-benzyl-4-(((2S)-2-((tert-butoxycarbonyl)amino)-1-cyano-3-(1H-indol-3-yl)propyl)-amino)-[1,1'-biphenyl]-3-carboxylate;
   2-(((2S)-2-(tert-butoxycarbonylamino)-1-cyano-3-(1H-indol-3-yl)propyl)amino)-N-benzyl-benzamide;
   (3S)-2-((2-bromophenyl)amino)-4-(1H-indol-3-yl)-3-(tert-butoxycarbonylamino) butanenitrile;
   methyl 2-(((2R)-2-((tert-butoxycarbonyl)amino)-1-cyano-3-(1H-indol-3-yl)propyl)amino)-benzoate;
   (3S)-3-(tert-butoxycarbonylamino)-2-((2-acetylphenyl)amino)-4-(1H-indol-3-yl)butanenitrile;
   methyl 4-(((2S)-2-((tert-butoxycarbonyl)amino)-1-cyano-3-(1H-indol-3-yl)propyl)amino)-benzoate;
   tert-butyl ((2S)-1-((2-benzoylphenyl)amino)-1-cyano-3-(1H-indol-3-yl)propan-2-yl)carbamate; and
   methyl 2-(((2S)-2-((tert-butoxycarbonyl)amino)-1-cyano-3-(1H-indol-3-yl)propyl)amino)benzoate

The compound of the first aspect of the invention may be comprised in a pharmaceutical composition. A second aspect of the invention thus relates to a composition comprising a compound of formula (I) as defined in the first aspect of the invention or a pharmaceutically acceptable salt thereof and one or more pharmaceutically acceptable diluent or carrier.

The compounds of formula (I) and their pharmaceutically acceptable salts are useful in the prevention or treatment of infection with coronavirus. A third aspect of the invention relates to a compound of formula (I) as defined in the first aspect of the invention or a pharmaceutically acceptable salt thereof, or a composition as defined in the second aspect of the invention, for use in medicine.

A fourth aspect of the invention relates to a compound of formula (I) as defined in the first aspect of the invention or a pharmaceutically acceptable salt thereof, or a composition as defined in the second aspect of the invention, for use in the treatment or prevention of viral infection by coronavirus.

A fifth aspect of the invention relates to a process for the preparation of a compound of formula (I) as defined in the first aspect of the invention wherein R⁴ is a hydrogen or an halogen atom, comprising the steps of:
(a) contacting a compound of formula (II) with a compound of formula (III) 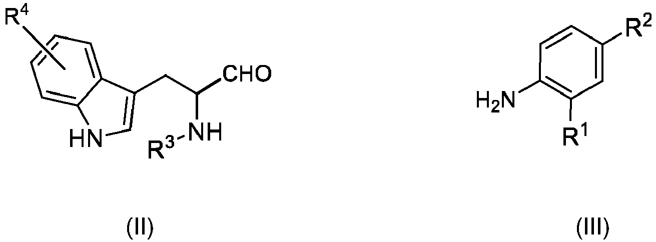 in order to form a compound of formula (IV) 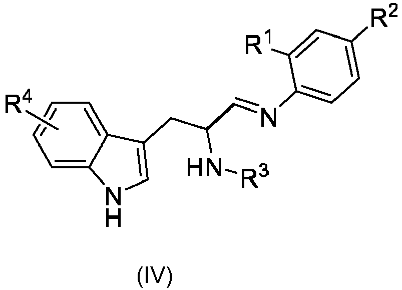 wherein R¹, R² and R³ and R⁴ are as defined in the first aspect of the invention, and
(b) treating the product of step (a) with a cyanide source in order to form a product of formula (I).

A sixth aspect of the invention relates to a process for the preparation of a compound of formula (I) as defined in the first aspect of the invention wherein R⁴ is a C₁₋₄-alkynylphenyl group, comprising the step of contacting the compound of formula (Ib) 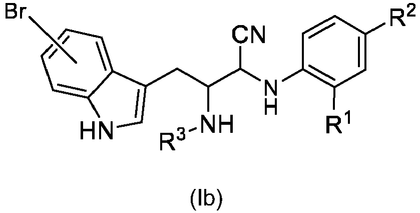 wherein R¹, R² and R³ are as defined for the compounds of formula (I) with a C₁₋₄-alkynylphenylboronic acid in the presence of a catalytically effective amount of a cross-coupling catalyst.

A seventh aspect of the invention relates to the use of a compound of formula (I) as defined in the first aspect of the invention or a pharmaceutically acceptable salt thereof, or a composition as defined in the second aspect of the invention, for the manufacture of a medicament for the treatment or prevention of viral infection by coronavirus.

An eighth aspect of the invention relates to a method of treating a subject suffering from a viral infection by coronavirus or at risk of suffering from a viral infection by coronavirus by administration to said subject of a compound of formula (I) as defined in the first aspect of the invention or a pharmaceutically acceptable salt thereof, or a composition as defined in the second aspect of the invention.

### DETAILED DESCRIPTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio, volume ratio and the like, should be considered approximate (i.e. with a 5% margin of variation around indicated point), unless specifically stated.

In the context of the invention, the term "alkyl" refers to an aliphatic saturated hydrocarbon chain that is linear or branched and having the number of carbon atoms defined in the claims and the description. Non-limiting examples of alkyl groups include, for instance, methyl, ethyl, *n*-propyl, *i*-propyl, *n*-butyl, *i*-butyl, *t*-butyl, *n*-pentyl, neo-pentyl, hexyl, heptyl, octyl, nonyl, decyl, undecanyl and dodecanyl.

In the context of the invention, the term "halogen" or "halo" refers to a halogen group, such as chloro, bromo, fluoro and iodo.

In the context of the invention, the term "cycloalkyl" refers to an aliphatic saturated hydrocarbon cycle and having the number of carbon atoms defined in the claims and the description. Non-limiting examples of cycloalkyl groups include, for instance, cyclopropyl, cyclobutyl, cyclopentyl and cyclohexyl.

In the context of the invention, the term "haloalkyl" refers to an alkyl group as defined above and having the number of carbon atoms defined in the claims and the description whereby at least one of the hydrogen atoms is replaced with a halo group. Thus, non-limiting examples of haloalkyl groups include, among others, chloromethyl, fluoromethyl, perfluoroalkyl groups such as trifluoromethyl, tetrafluoroethyl, and bromomethyl.

In the context of the invention, the term "alkylcarbonyl" refers to a saturated linear or branched hydrocarbon group having the number of carbon atoms indicated in the description or in the claims which is attached to the remainder of the molecular formula through a carbonyl group (C=O).

In the context of the invention, the term "alkylcarbonyloxy" refers to a saturated linear or branched hydrocarbon group having the number of carbon atoms indicated in the description or in the claims which is attached to the remainder of the molecular formula through a carboxyl group (-COO-) and wherein the alkyl chain is attached to the carbon atom of the carboxyl group.

In the context of the invention, the term "alkyloxy" refers to a saturated linear or branched hydrocarbon group having the number of carbon atoms indicated in the description or in the claims which is attached to the remainder of the molecular formula through a oxy group (-O-) and wherein the alkyl chain is attached to the oxygen atom of the oxy group.

In the context of the invention, the term "alkyloxycarbonyl" refers to a saturated linear or branched hydrocarbon group having the number of carbon atoms indicated in the description or in the claims which is attached to the remainder of the molecular formula through a carboxyl group (-OOC-) and wherein the alkyl chain is attached to the oxygen atom of the carboxyl group.

In the context of the invention, the term "heterocycle" or also "heterocyclic ring" refers to a monocyclic or bycyclic group having carbon atoms and at least one heteroatom selected from the group consisting of oxygen, sulfur and nitrogen as part of the cycle.

In context of the invention, the term "saturated" when used in relation with a heterocycle is meant to indicate that said heterocycle contains no unsaturations (double or triple bonds) as part of the heterocycle.

In context of the invention, the term "unsaturated" when used in relation with a heterocycle is meant to indicate that said heterocycle contains at least one unsaturation (double or triple bonds) as part of the heterocycle. An unsaturated heterocycle might be aromatic or non-aromatic.

In context of the invention, the term "acetamido" is used to indicate the group CH₃-CONH-.

In context of the invention, the term "acetyl" is used to indicate the group CH₃-CO-.

In the context of the invention, the term "catalytically effective amount" refers to the fact that the amount of catalyst is much smaller than the stoichiometric amounts of either starting materials of a catalytic reaction and is sufficient for the catalyzed process to take place at a reaction rate that is at least twice the rate of the non- catalyzed process.

As defined above, a first aspect of the invention relates to a compound of formula (I) as defined above in the first aspect of the invention.

In a preferred embodiment of the first aspect of the invention, the compound of formula (I) is as defined in the first aspect of the invention with the proviso that when R⁴ is hydrogen and R¹ is -COO-C₁₋₄alkyl, CH₃-CO-, benzoyl or -CO-NH-CH₂-phenyl, then R³ is not one of 2,3-dihydro-benzo[1,4]dioxin-6-yl, 2-ethoxy-pyrimidin-5-yl, 2-fluoro-5-isopropyloxyphenyl, 2-morpholinopyridin-4-yl, 3-(N-pyrrolidinyl)phenyl, 3-benzylphenyl, 3-diethylaminophenyl,3-ethylphenyl, 3-N-(morpholinyl)phenyl, 3-tert-butylphenyl, 3-trifluoromethyloxy-phenyl, 4-(4-methylpiperazin-1-yl)phenyl, 4-butylphenyl, 4-cyclopropylphenyl, 4-diethylaminophenyl, 4-ethylphenyl, 4-N-(morpholinyl)phenyl, 4-propoxyphenyl, 4-tert-butylphenyl, 6-(4-isopropylpiperazin-1-yl)pyridin-3-yl, 6-(N-morpholinyl)pyridine-3-yl, 6-methoxy-pyridin-3-yl, 6-methyl-pyridin-3-yl, 6-pyrrolidin-pyridin-3-yl, benzyl, methoxycarbonyl, Br, Cl ,CO₂Me and H.

The compound of formula (I) may exist in different diastereomeric forms, in particular, the following diastereomeric forms (I'a), (I'b), (I"a) and (I"b) are contemplated:

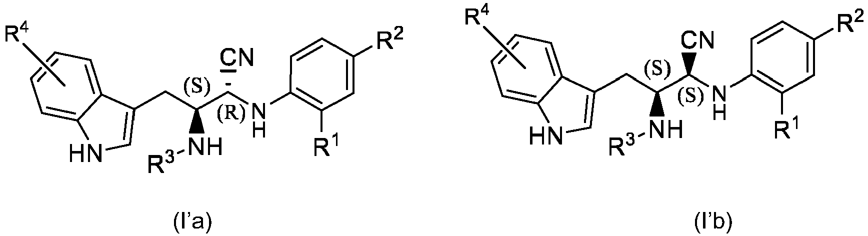

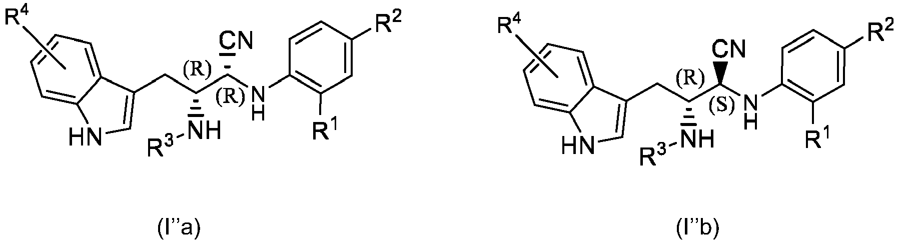

As disclosed herein, when the configuration of a stereocenter in the compound of formula (I) is not specified or is represented by a coplanar bond, it refers to the fact that said stereocenter may be in either (R) or (S) configuration. Thus, formula (I) with non-specified configuration of the two stereocenters is intended to cover the 4 possible stereoisomers (R,R), (R,S), (S,R) and (S,S) or any mixture thereof. In a particular embodiment the compound may exist as a mixture of two compounds whereby, in the first compound, said stereocenter is in the (R) configuration and, in the second compound, said stereocenter is in the (S) configuration.

In a preferred embodiment of the various aspects of the invention, in the compound of formula (I) the group R¹ is methyloxycarbonyl.

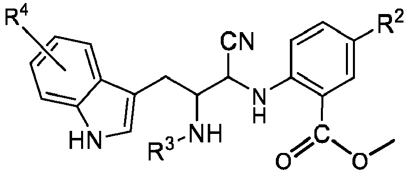

In a preferred embodiment of the first aspect of the invention, the compound of formula (I) is one of formula (I') or a pharmaceutically acceptable salt thereof

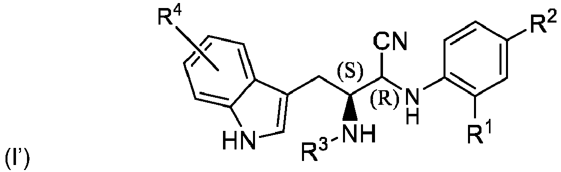

The compound of formula (I') thus represents a compound of formula (I'a) as defined above or a compound of formula (I'b) as defined above or a mixture thereof.

In a more preferred embodiment of the first aspect of the invention, the compound of formula (I) is a mixture of the diastereomeric forms (I'a) and (I'b) as defined above.

In particular embodiments of the first aspect of the invention, when the compound of formula (I) is a mixture of the diastereomeric forms (I'a) and (I'b) as defined above, the molar ratio of the compound of formula (I'a) to the compound of formula (I'b) is comprised from 1:10 to 10:1.

In particular embodiments of the first aspect of the invention, when the compound of formula (I) is a mixture of the diastereomeric forms (I'a) and (I'b) as defined above, the molar ratio of the compound of formula (I'a) to the compound of formula (I'b) is comprised from 1:5 to 10:1.

In more particular embodiments of the first aspect of the invention, when the compound of formula (I) is a mixture of the diastereomeric forms (I'a) and (I'b) as defined above, the molar ratio of the compound of formula (I'a) to the compound of formula (I'b) is selected from the group consisting of 9:1, 2:1, 1:1, 1:1.3, 1:1.5, 1:2, 1:3 and 1:5.

In other preferred embodiments of the first aspect of the invention, the compound of formula (I) is one wherein R² is a cycle selected from the group consisting of pyridinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group -G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom; wherein m represents and integer selected from 0, 1 and 2.

In another preferred embodiment of the first aspect of the invention, the compound of formula (I) is one in which R⁴ is a group selected from the group consisting of hydrogen, bromine, 4-ethynylphenyl and 4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl.

The different groups R¹, R², R³ and R⁴ defined in the particular and preferred embodiments above may be combined to provide compounds of formula (I) according to the first aspect of the invention.

Particularly, the compound of the first aspect of the invention is selected from the group consisting of:
Ex. 1: Methyl 4-(((1RS,2S)-3-(5-bromo-1H-indol-3-yl)-2-((tert-butoxycarbonyl)amino)-1-cyanopropyl)amino)-4'-morpholino-[1,1'-biphenyl]-3-carboxylate
Ex. 2: Methyl 4-(((1RS,2S)-3-(6-bromo-1H-indol-3-yl)-2-((tert-butoxycarbonyl)amino)-1-cyanopropyl)amino)-4'-morpholino-[1,1'-biphenyl]-3-carboxylate
Ex. 3: Methyl 4-(((1RS,2S)-3-(7-bromo-1H-indol-3-yl)-2-((tert-butoxycarbonyl)amino)-1-cyanopropyl)amino)-4'-morpholino-[1,1'-biphenyl]-3-carboxylate Ex. 4: Methyl 4-(((1RS,2S)-3-(4-bromo-1H-indol-3-yl)-2-((tert-butoxycarbonyl)amino)-1-cyanopropyl)amino)-4'-morpholino-[1,1'-biphenyl]-3-carboxylate
Ex. 5: Methyl 4-(((1RS,2S)-2-((tert-butoxycarbonyl)amino)-1-cyano-3-(5-phenyl-1H-indol-3-yl)propyl)amino)-4'-morpholino-[1,1'-biphenyl]-3-carboxylate
Ex. 6: Methyl 4-(((1RS,2S)-2-((tert-butoxycarbonyl)amino)-1-cyano-3-(4-(4-ethynylphenyl)-1H-indol-3-yl)propyl)amino)-4'-morpholino-[1,1'-biphenyl]-3-carboxylate
Ex. 7: Methyl 2-(((1RS,2S)-2-((tert-butoxycarbonyl)amino)-1-cyano-3-(1H-indol-3-yl)propyl)amino)-5-(2-morpholinopyridin-4-yl)benzoate
Ex. 8: Methyl 4-(((1RS,2S)-2-((tert-butoxycarbonyl)amino)-1-cyano-3-(1H-indol-3-yl)propyl)amino)-4'-(morpholine-4-carbonyl)-[1,1'-biphenyl]-3-carboxylate
Ex. 9: Methyl 2-(((1RS,2S)-2-((tert-butoxycarbonyl)amino)-1-cyano-3-(1H-indol-3-yl)propyl)amino)-5-(1H-indol-5-yl)benzoate
Ex. 10: Methyl 4-(((1RS,2S)-2-((tert-butoxycarbonyl)amino)-1-cyano-3-(1H-indol-3-yl)propyl)amino)-4'-(morpholinomethyl)-[1,1'-biphenyl]-3-carboxylate
Ex. 11: Methyl 2-(((1RS,2S)-2-((tert-butoxycarbonyl)amino)-1-cyano-3-(1H-indol-3-yl)propyl)amino)-5-(1H-indol-6-yl)benzoate
Ex. 12: Methyl 5-(benzofuran-2-yl)-2-(((1RS,2S)-2-((tert-butoxycarbonyl)amino)-1-cyano-3-(1H-indol-3-yl)propyl)amino)benzoate
Ex. 13: Methyl 5-(benzo[b]thiophen-2-yl)-2-(((1RS,2S)-2-((tert-butoxycarbonyl)amino)-1-cyano-3-(1H-indol-3-yl)propyl)amino)benzoate
Ex. 14: tert-Butyl 4-(4-(((1RS,2S)-2-((tert-butoxycarbonyl)amino)-1-cyano-3-(1H-indol-3-yl)propyl)amino)-3-(methoxycarbonyl)phenyl)-3,6-dihydropyridine-1(2H)-carboxylate Ex. 15: Methyl 5-(1H-benzo[d]imidazol-5-yl)-2-(((1RS,2S)-2-((tert-butoxycarbonyl) amino)-1-cyano-3-(1H-indol-3-yl)propyl)amino)benzoate
Ex. 16: Methyl 2-(((1RS,2S)-2-((tert-butoxycarbonyl)amino)-1-cyano-3-(1H-indol-3-yl)propyl)amino)-5-(1H-indazol-5-yl)benzoate
Ex. 17: Methyl 5-(1-benzyl-1H-pyrazol-4-yl)-2-(((1RS,2S)-2-((tert-butoxycarbonyl) amino)-1-cyano-3-(1H-indol-3-yl)propyl)amino)benzoate
Ex. 18: Methyl 2-(((1RS,2S)-2-((tert-butoxycarbonyl)amino)-1-cyano-3-(1H-indol-3-yl)propyl)amino)-5-(1H-pyrrolo[2,3-b]pyridin-3-yl)benzoate
Ex. 19: tert-Butyl 5-(4-(((1RS,2S)-2-((tert-butoxycarbonyl)amino)-1-cyano-3-(1H-indol-3-yl)propyl)amino)-3-(methoxycarbonyl)phenyl)-1H-indole-1-carboxylate
Ex. 20: Methyl 4-(((1RS,2S)-2-((tert-butoxycarbonyl)amino)-1-cyano-3-(4-(4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl)-1H-indol-3-yl)propyl)amino)-4'-morpholino-[1,1'-biphenyl]-3-carboxylate
Ex. 21: Methyl 4-(((1RS,2S)-2-((tert-butoxycarbonyl)amino)-1-cyano-3-(1H-indol-3-yl)propyl)amino)-4'-fluoro-3'-(2H-tetrazol-5-yl)-[1,1'-biphenyl]-3-carboxylate Ex. 22: Methyl 4-(((1RS,2S)-2-((tert-butoxycarbonyl)amino)-1-cyano-3-(1H-indol-3-yl)propyl)amino)-2',3',4',5'-tetrahydro-[1,1'-biphenyl]-3-carboxylate Ex. 23: Methyl 2-(((1RS,2S)-2-((tert-butoxycarbonyl)amino)-1-cyano-3-(1H-indol-3-yl)propyl)amino)-5-(1-(tetrahydro-2H-pyran-2-yl)-1H-pyrazol-5-yl)benzoate Ex. 24: Methyl 5-(benzo[d]oxazol-5-yl)-2-(((1RS,2S)-2-((tert-butoxycarbonyl)amino)-1-cyano-3-(1H-indol-3-yl)propyl)amino)benzoate
Ex. 25: Methyl 2-(((1RS,2S)-2-((tert-butoxycarbonyl)amino)-1-cyano-3-(1H-indol-3-yl)propyl)amino)-5-(1-(2-morpholinoethyl)-1H-pyrazol-4-yl)benzoate Ex. 26: tert-Butyl ((1RS,2R)-1-((2-benzoyl-4-bromophenyl)amino)-1-cyano-3-(1H-indol-3-yl)propan-2-yl)carbamate
Ex. 27: Methyl 2-(((1RS,2R)-2-((tert-butoxycarbonyl)amino)-1-cyano-3-(1H-indol-3-yl)propyl)amino)-5-(1H-indol-5-yl)benzoate
Ex. 28: Methyl 2-(((1RS,2R)-2-((tert-butoxycarbonyl)amino)-1-cyano-3-(1H-indol-3-yl)propyl)amino)-5-(2,3-dihydrobenzo[b][1,4]dioxin-6-yl)benzoate Ex. 29: Methyl 5-(6-acetamidopyridin-3-yl)-2-(((1RS,2S)-2-((tert-butoxycarbonyl)amino)-1-cyano-3-(1H-indol-3-yl)propyl)amino)benzoate
Ex. 30: Methyl 2-(((1RS,2S)-2-((tert-butoxycarbonyl)amino)-1-cyano-3-(1H-indol-3-yl)propyl)amino)-5-(1H-indazol-4-yl)benzoate
Ex. 31: tert-Butyl ((1RS,2S)-1-((2-(azepane-1-carbonyl)phenyl)amino)-1-cyano-3-(1H-indol-3-yl)propan-2-yl)carbamate

As further detailed in the Examples below, these compounds are particularly active in the prevention or treatment of infection with coronavirus.

The compound of formula (I) of the first aspect of the invention may also be in the form of a pharmaceutically acceptable salt. Suitable anions forming pharmaceutically acceptable salts are known in the art and include, for instance, aspartate, benzenesulfonate, benzoate, besylate, bicarbonate, bitartrate, bromide, camsylate, carbonate, chloride, citrate, decanoate, edetate, esylate, fumarate, gluceptate, gluconate, glutamate, glycolate, hexanoate, hydroxynaphthoate, iodide, isethionate, lactate lactobionate, malate, maleate, mandelate, mesylate, methylsulfate, mucate, napsylate, nitrate, octanoate, oleate, pamoate, pantothenate, phosphate, polygalacturonate, propionate, salicylate, stearate, acetate, succinate, sulfate, tartrate, teoclate, and tosylate.

Suitable cations forming pharmaceutically acceptable salts are known in the art and include, for instance, aluminium, argininium, calcium, lithium, potassium, sodium and zinc.

As detailed above, the second aspect of the invention relates to a composition comprising a compound of formula (I) as defined in any of the particular and preferred embodiments of the first aspect of the invention defined above, or a pharmaceutically acceptable salt thereof and one or more pharmaceutically acceptable diluent or carrier.

The composition according to the present invention may be in any form suitable for the application to humans and/or animals, preferably humans including infants, children and adults, and can be produced by standard procedures known to those skilled in the art. Therefore, the composition in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes, such as spray injection or infusion.

The composition according to the second aspect of the invention may further be transferred into usual formulations, such as tablets, sugar-coated tablets, pills, granules, aerosols, syrups, emulsions, suspensions, solutions, ointments, creams, dermal patches and gel of any kind, using in particular essentially non-toxic and pharmaceutically acceptable carriers and diluents.

The composition of the second aspect of the invention may in certain embodiments further comprise auxiliary materials or additives of a pharmaceutical composition selected among excipients, support materials, lubricants, fillers, solvents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition and will become apparent to the skilled person.

In an embodiment the pharmaceutical compositions of the second aspect of the invention are a form suitable for oral administration, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The composition of the second aspect may also be adapted for parenteral administration, such as sterile solutions, suspensions or reconstitutable dry preparations, aerosols or sprays in the appropriate unit dosage form. Adequate excipients can be used, such as bulking agents, buffering agents or surfactants.

In a preferred embodiment of the second aspect, the composition comprises a therapeutically effective amount of the compound of formula (I) as defined in the first aspect of the invention. The physician will determine the dosage of the present therapeutic agents which will be most suitable and it will vary with the form of administration and the particular compound chosen, and furthermore, it will vary with the patient under treatment, the age of the patient, the type of disease or condition being treated. For instance, when the composition is administered orally, larger quantities of the active agent will be required to produce the same effect as a smaller quantity given parenterally. The compounds are useful in the same manner as comparable anti-infective agents and the dosage level is of the same order of magnitude as is generally employed with these other anti-infective agents.

The compounds and compositions of this invention may be used with other drugs to provide a combination therapy. The other drugs may form part of the same composition, or be provided as a separate composition for administration at the same time or at different time. The compound of formula (I) may thus be combined with other anti-viral agents, such as remdesivir, nitazoxanide, nirmatrelvir, ivermectin and molnupiravir, as well as with antibodies having anti-viral activity.

As defined above, the inventors have found that the compounds of formula (I) are particularly useful in the prevention or treatment of infection with coronavirus.

A third aspect of the invention thus relates to a compound of formula (I) or a compound of formula (I1) as defined in the first aspect of the invention, or a pharmaceutically acceptable salt thereof, or a composition as defined in the second aspect of the invention for use in medicine.

A fourth aspect of the invention relates to a compound of formula (I) or a compound of formula (I1) as defined in the first aspect of the invention, or a pharmaceutically acceptable salt thereof, or a composition as defined in the second aspect of the invention for use in in the treatment or prevention of viral infection by coronavirus.

The fourth aspect of the invention also relates to a method of prevention or treatment of infection with a coronavirus, said method comprising the step of administering a therapeutically active amount of a compound of formula (I) as defined in the first aspect of the invention, or of a pharmaceutically acceptable salt thereof, or of a composition as defined in the second aspect of the invention to a patient susceptible of being or being infected with coronavirus.

The fourth aspect of the invention also relates to the use of a compound of formula (I) as defined in the first aspect of the invention, or of a pharmaceutically acceptable salt thereof, or of a composition as defined in the second aspect of the invention in the prevention or treatment of infection with coronavirus.

A process for the preparation of a compound of formula (I) is also part of the invention.

Thus, the fifth aspect of the invention relates to a process for the preparation of a compound of formula (I) as defined in the first aspect of the invention comprising the steps of:
(a) contacting a compound of formula (II) with a compound of formula (III) 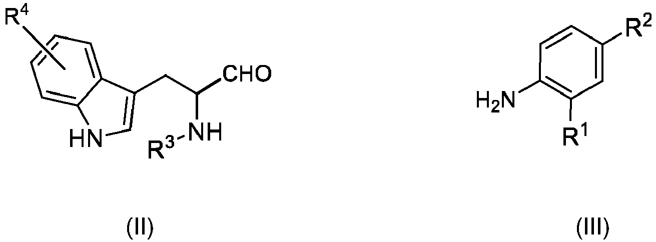 in order to form a compound of formula (IV) 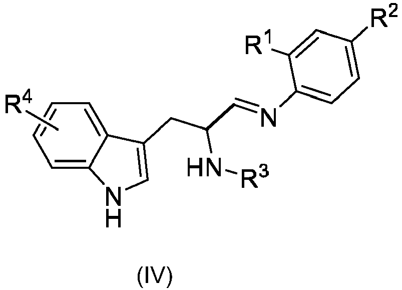 wherein R¹, R², R³ and R⁴ are as defined in the first aspect of the invention and (b) treating the product of step (a) with a cyanide source in order to form a product of formula (I).

In a preferred embodiment of the fifth aspect of the invention, the cyanide source of step (b) is selected from the group consisting of trimethylsilyl cyanide and potassium cyanide. Trimethylsilyl cyanide is a preferred cyanide source, because it is soluble in organic solvents such as methanol and ethanol.

In a preferred embodiment of the fifth aspect of the invention, steps (a) and (b) are carried out without isolating the compound of formula (IV).

In a preferred embodiment of the fifth aspect of the invention, step (a) is carried out by heating a solution of the compounds of formulae (II) and (III) in the presence of a Lewis acid, such as zinc(II) chloride. A suitable temperature may be between 50 °C and 100 °C. Alternative conditions for the formation of an imine functional group by condensation of an amine with an aldehyde, are known in the art and will become apparent to the skilled person.

In a preferred embodiment of the fifth aspect of the invention, step (b) is carried out at room temperature.

In a preferred embodiment of the fifth aspect of the invention, each of steps (a) and (b) are carried out in methanol.

In a preferred embodiment, the process of the fifth aspect of the invention comprises the previous step of preparing the compound of formula (II) by treating a compound of formula (V) with a hydride source

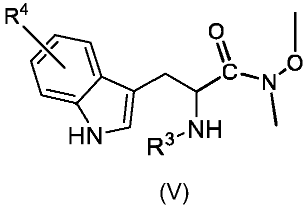

The hydride source of said previous step is preferably lithium aluminium hydride.

In a preferred embodiment, the process of the fifth aspect of the invention comprises the previous step of preparing the compound of formula (V) by contacting a compound of formula (VI) with N,O,-methoxidimethylhydroxylamine in the presence of a peptide coupling agent.

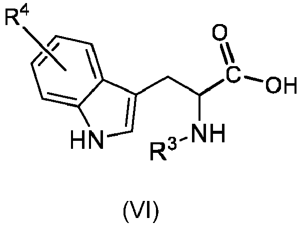

Suitable peptide coupling agents are well known in the art and will become apparent to the skilled person. For instance, a suitable peptide coupling agent may be N,N'-diisopropylcarbodiimide combined with a base, such as 4-dimethylaminopyridine (DMAP).

In a particular embodiment of the fifth aspect of the invention, the process of the fifth aspect of the invention comprises the steps of:
a) contacting a compound of formula (II) with a compound of formula (Illa) 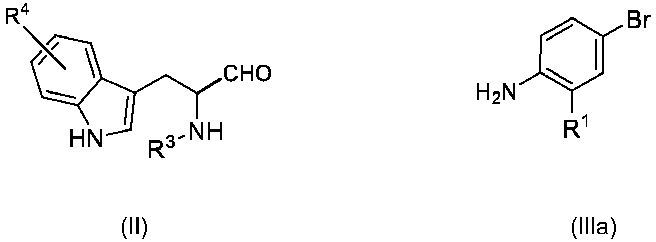 in order to form a compound of formula (IVa) 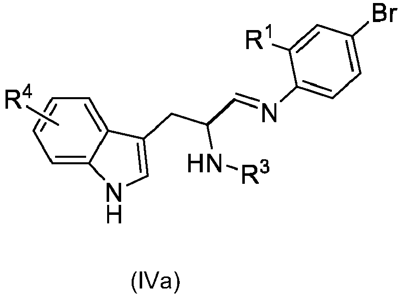 wherein R¹, R³ and R⁴ are as defined in the first aspect of the invention, and (b) treating the product of step (a) with a cyanide source in order to form a product of formula (Ia). 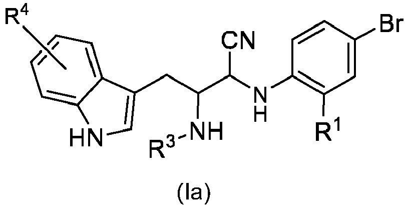 and (c) the step of contacting the compound of formula (Ia) with a compound of formula R²-B(OH)₂ or with a compound of formula 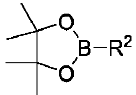 in the presence of a catalytically effective amount of a cross-coupling catalyst, wherein R² is a group selected from a cycle selected from the group consisting of pyridinyl, pyrimidinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, 2,3-dihydrobenzo[b][1,4]dioxine and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group - G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom;

Said further step of cross-coupling is preferably carried out in the presence of a base. Suitable bases for cross-coupling reactions, such as Suzuki couplings, are well known in the art and will become apparent to the skilled person. It is preferred that the base is an alkaline carbonate salt, such as potassium carbonate or an alkaline phosphate, such as potassium phosphate.

Suitable catalysts for cross-coupling reactions, such as Suzuki couplings, are well known in the art and will become apparent to the skilled person. It is preferred that said catalyst is a palladium(0) catalyst, such as tetrakis(triphenylphosphine)palladium or a palladium(II) catalyst, such as chloro(2-dicyclohexylphosphino-2',4',6'-triisopropyl-1,1'-biphenyl)[2-(2'-amino-1,1'-biphenyl)]palladium(II) (XPhos-Pd-G2).

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

A sixth aspect of the invention relates to a process for the preparation of a compound of formula (I) as defined in the first aspect of the invention wherein R⁴ is a C₁₋₄-alkynylphenyl group, comprising the step of contacting the compound of formula (Ib) 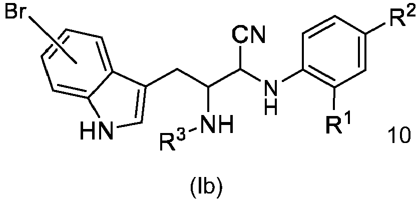 wherein R¹, R² and R³ are as defined for the compounds of formula (I) with a C₁₋₄-alkynylphenylboronic acid in the presence of a catalytically effective amount of a cross-coupling catalyst. The compounds of formula (Ib) may be prepared according to the fifth aspect of the invention described above.

Said further step of cross-coupling is preferably carried out in the presence of a base. Suitable bases for cross-coupling reactions, such as Suzuki couplings, are well known in the art and will become apparent to the skilled person. It is preferred that the base is an alkaline carbonate salt, such as potassium carbonate or an alkaline phosphate, such as potassium phosphate.

Suitable catalysts for cross-coupling reactions, such as Suzuki couplings, are well known in the art and will become apparent to the skilled person. It is preferred that said catalyst is a palladium(0) catalyst, such as tetrakis(triphenylphosphine)palladium or a palladium(II) catalyst, such as chloro(2-dicyclohexylphosphino-2',4',6'-triisopropyl-1,1'-biphenyl)[2-(2'-amino-1,1'-biphenyl)]palladium(II) (XPhos-Pd-G2).

### EXAMPLES

### General considerations

Reactions were monitored either by TLC and/or analytic HPLC (UV, detection, 220 nm). Flash columns, filled with silica gel Merck 60 (230-400) were used for chromatographic separations. A reversed-phase column, Sunfire C18 (4.6 × 50 mm, 3.5 µm), a flux of 1 mL/min, and mixtures of CH₃CN, phase A, and H₂O, phase B, both containing 0.01% formic acid, were used for analytical HPLCs. Mass spectra, in electrospray, positive mode, were obtained on a Waters Micromass ZQ spectrometer. The peaks corresponding to the molecular ion have been indicated as ([M]⁺), while those corresponding to the molecular ion plus one proton as ([M⁺H]⁺). NMR spectra were recorded in a Varian INOVA-400 (400 MHz) spectrometer, operating at 400 and 100 MHz for 1H and ¹³C experiments, respectively (with chemical shifts expressed in ppm and coupling constants in Hz).

### SYNTHESIS OF COMPOUNDS OF FORMULA (I)

Step c) of the process according to the fifth aspect of the present invention as well as the process according to the sixth aspect of the present invention may be carried out in particular following various detailed protocols (A and B) explained below:

### Protocol A)

An aryl halide derivative of formula (1.0 equiv), the corresponding boronic acid (1.5 equiv) and K₂CO₃ (6 equiv) is dissolved in THF/H₂O (5 mL/mmol , 4/1) in a microwave vial. Argon is bubbled for 10 min., then [Pd(PPh₃)₄] (2%) is added under a positive pressure of Argon. The vial is sealed, evacuated and backfilled with Argon, and then irradiated in a microwave apparatus at 100 °C, for 30 min. After the reaction mixture is cooled to room temperature, the solvent is evaporated to dryness. The resulting solid is resuspended in water (10 mL) and the aqueous phase is extracted with EtOAc (3x10 mL). The combining organic phases are dried over Na₂SO₄, filtrated and evaporated to dryness. The crude mixture is purified by flash chromatography using the appropriate eluent system. The examples indicate the eluent system used for each of them.

### Protocol B)

The corresponding boronic pinacol ester is added to a microwave flask (1.0 equiv), alongside triphenylphosphine palladium tetrakis (8% mol.). The flask is sealed and a solution in 1,4-dioxane of the bromo-derivative product of the Strecker reaction (1.0 equiv) (0.1M) is added. Afterwards, an aqueous solution of Na₂CO₃ 2M is added (0.1 ml / 1 ml solvent) and the final solution is bubbled with argon. The flask is left reacting in the microwave (140°C, 30 min). After the reaction mixture is cooled to room temperature, the solvent is evaporated to dryness. The resulting solid is resuspended in water (10 mL) and the aqueous phase is extracted with EtOAc (3x10 mL). The crude mixture is purified by flash chromatography using the appropriate eluent system. The examples indicate the eluent system used for each of them.

The compounds of the present invention in which R⁴ is 4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl may be prepared from the corresponding compound wherein R4 is ethynylphenyl following a click type reaction as described in protocol C below:

### Protocol C)

The corresponding alkyne (1.02 equiv), CuSO₄·H₂O (1.0 equiv), L-sodium ascorbate (2 equiv), Biotin-PEG3-azide (1.0 equiv) and a magnetic stirring bar are added to a reaction flask. 1 mL of DMSO are added. The mixture is stirred and warmed to 40 °C for 21 hours. After the reaction mixture is cooled to room temperature, AcOEt and brine are added, and the organic layer is separated. The aqueous layer is further extracted with AcOEt, and the combined organic layer is washed with brine, dried over Na₂SO₄, filtered and evaporated *in vacuo.* The reaction is lyophilized to eliminate DMSO. The crude mixture is purified by flash chromatography using the appropriate eluent system. The examples indicate the eluent system used for each of them.

The process according to steps a) and b) of the fifth aspect of the present invention may be carried out in particular following various detailed protocols (D and E) explained below:

### Protocol D)

The corresponding amine compound of formula (ilia) (2 mmol) and ZnCl₂ (1.1 mmol) are added to a solution of the compound of formula (IIa) (1 mmol) in MeOH (20 mL), and the mixture is refluxed for 12 h. After this time, the reaction mixture is allowed to cool at room temperature, and trimethylsilyl cyanide (TMSCN) (1.09 mmol) is added. After stirring for 24 h at room temperature, the solvent is evaporated and the residue was dissolved in EtOAc (20 mL). This solution is successively washed with H₂O (2x5 mL), brine (5 mL), dried over Na₂SO₄ and evaporated to dryness. The crude mixture is purified by flash chromatography using the appropriate eluent system. The examples indicate the eluent system used for each of them.

### Protocol E)

The corresponding amine compound of formula (III) (2 mmol) and ZnCl₂ (1.1 mmol) are added to a solution of the corresponding aminoaldehyde compound of formula (II) (1 mmol) in MeOH (20 mL), and the mixture is refluxed for 12 h. After this time, the reaction mixture is allowed to cool to room temperature and trimethylsilyl cyanide (TMSCN) (1.09 mmol) is added. After stirring for 24 h at room temperature, the solvent is evaporated and the residue is dissolved in EtOAc (20 mL). This solution is washed successively with H₂O (2x5 mL), brine (5 mL), dried over Na₂SO₄ and evaporated to dryness. The crude mixture is purified by flash chromatography using the appropriate eluent system. The examples indicate the eluent system used for each of them.

The amine compound of formula (III) may be obtained by a Suzuki reaction as described in Protocol A or Protocol B.

### EXAMPLES

### Example 1:

### Methyl 4-(((1RS,2S)-3-(5-bromo-1H-indol-3-yl)-2-((tert-butoxycarbonyl)amino)-1-cyanopropyl)amino)-4'-morpholino-[1,1'-biphenyl]-3-carboxylate

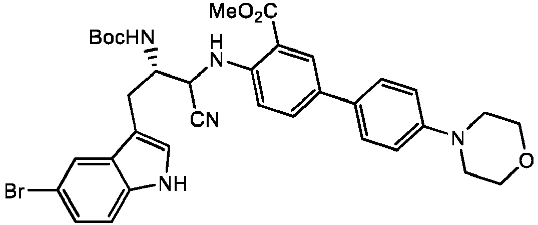

C₃₅H₃₈N₄BrN₅O₅, 688.62 g·mol⁻¹

Obtained following Protocol A and Protocol E.

Purification by flash chromatography, using 0% to 50% of EtOAc in hexane gradient as eluants, furnished the desired compound as an epimeric mixture, ratio 1:3/*1R*,*2S*:*1S*,*2S* in 53 % yield
¹H-NMR (400 MHz, CDCl₃) δ (ppm): 8.34 (d, *J* = 8.6 Hz, 1.33H), 8.23 (s, 1H), 8.18 (d, *J* = 2.4 Hz, 0.33H), 8.16 (d, *J* = 2.3 Hz, 1H), 7.79 (s, 1H), 7.66 (dd, *J* = 10.8, 2.1 Hz, 0.33H), 7.59 (dd, *J* = 8.6, 2.4 Hz, 1.33H), 7.47 (t, *J* = 8.9 Hz, 2.66H), 7.32 (d, *J* = 1.8 Hz, 0.33H), 7.30 (d, *J* = 1.8 Hz, 1H), 7.26 (d, *J=* 8.7 Hz, 3.66H), 7.16 (d, *J* = 2.6 Hz, 1.33H), 6.97 (d, *J* = 8.3 Hz, 2.66H), 6.63 (d, *J* = 8.9 Hz, 1.33H), 4.90 (d, *J* = 9.7 Hz, 1H), 4.81 (s, 0.33H), 4.59 (s, 1H), 4.50 - 4.37 (m, 2H), 3.92 (s, 1H), 3.88 (d, *J* = 7.1 Hz, 9.5H), 3.39 - 3.23 (m, 1.66H), 3.19 (q, *J* = 4.8 Hz, 7H), 3.14 (d, *J =* 7.6 Hz, 0.66H), 1.49 (s, 9H), 1.43 (d, *J* = 2.3 Hz, 3H).

¹³C-NMR (100 MHz, CDCl₃) δ (ppm): 169.08, 168.77, 155.39, 150.38, 146.88, 146.60, 135.11, 132.97, 131.79, 130.55, 129.77, 129.11, 127.27, 125.69, 124.40, 121.54, 117.80, 117.50, 116.10, 113.52, 112.91, 112.54, 110.10, 110.03, 80.86, 77.36, 67.01, 52.55, 52.03, 49.47, 48.55, 41.16, 28.44, 28.18.

**HPLC** (Sunfire C18, 50-95% of A in B, 10 min): t_{R} = 7.8 min.

**LC-MS (m/z):** 690.25 ([M+H]⁺).

### Example 2:

### Methyl 4-(((1RS,2S)-3-(6-bromo-1H-indol-3-yl)-2-((tert-butoxycarbonyl)amino)-1-cyanopropyl)amino)-4'-morpholino-[1,1'-biphenyl]-3-carboxylate

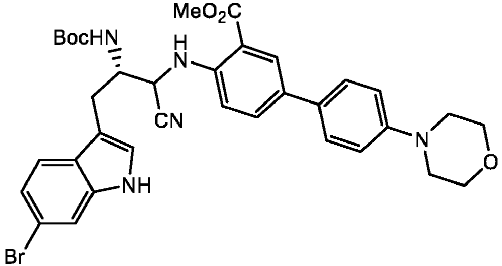

C₃₅H₃₈N₄BrN₅O₅, 688.62 g·mol⁻¹

Obtained following Protocol A and Protocol E.

Purification by flash chromatography, using 5% to 50% of EtOAc in hexane gradient as eluants, furnished the desired compound as an epimeric mixture, ratio 1:3/*1R*,*2S*:*1S*,*2S* in 53 % yield.

¹H-NMR (400 MHz, CDCl₃) δ (ppm): 8.32 (d, *J* = 8.6 Hz, 1H), 8.27 (d, *J* = 7.9 Hz, 0.33H), 8.21 (s, 0.33H), 8.17 (d, *J* = 2.3 Hz, 0.33H), 8.15 (d, *J* = 2.3 Hz, 1H), 7.66 (dd, *J* = 8.6, 2.4 Hz, 0.33H), 7.57 (s, 0.66H), 7.54 (d, *J* = 1.6 Hz, 2H), 7.52 (d, *J* = 1.6 Hz, 0.33H), 7.50 - 7.40 (m, 2H), 7.26 - 7.22 (m, 1H), 7.19 (dd, *J* = 8.4, 1.7 Hz, 0.33H), 7.14 (s, 0.66H), 7.07 (d, *J* = 2.6 Hz, 0.33H), 6.97 (t, *J* = 8.3 Hz, 2.66H), 6.58 (d, *J* = 8.7 Hz, 1H), 4.92 (d, *J* = 10.0 Hz, 1H), 4.80 (s, 0.66H), 4.61 (s, 1.33H), 4.48 - 4.40 (m, 1.66H), 3.90 (s, 1.33H), 3.89 (s, 1.66H), 3.88 (s, 8H), 3.38 - 3.24 (m, 1.66H), 3.20 (q, *J* = 5.1 Hz, 8H), 1.50 (s, 9H), 1.42 (s, 3H).

¹³C-NMR (100 MHz, CDCl₃) δ (ppm): 168.65, 155.50, 146.76, 137.28, 132.92, 129.85, 127.41, 126.16, 123.73, 123.48, 123.33, 120.19, 117.45, 116.34, 114.48, 112.88, 112.54, 110.53, 80.92, 77.36, 66.55, 52.62, 52.14, 52.02, 48.66, 48.40, 28.44.

**HPLC** (Sunfire C18, 80-95% of A in B, 5 min): t_{R} = 1.44 min.

**LC-MS (m/z):** 690.25 ([M+H]⁺).

### Example 3:

### Methyl 4-(((1RS,2S)-3-(7-bromo-1H-indol-3-yl)-2-((tert-butoxycarbonyl)amino)-1-cyanopropyl)amino)-4'-morpholino-[1,1'-biphenyl]-3-carboxylate

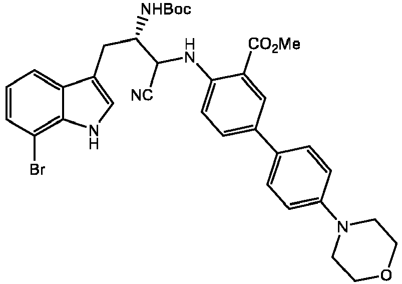

C₃₅H₃₃BrN₅O₅, 687.21 g.mol-1

Obtained following Protocol A and Protocol E.

Purification by flash chromatography, using 0% to 50% of EtOAc in hexane gradient as eluants, furnished the desired compound as an epimeric mixture, ratio 1:3/*1R,2S:1S,2S* in 52 % yield
¹H-NMR (400 MHz, CDCl₃) δ (ppm): 8.34 (s, 1.66H), 8.31 (s, 0.33H), 8.15 (d, J = 2.3 Hz, 1.33H), 7.65 (d, J = 7.6 Hz, 1.33H), 7.56 (dd, J = 8.5, 2.1 Hz, 1.33H), 7.53 - 7.46 (m, 2H), 7.45 (d, J = 8.8 Hz, 2.33H), 7.38 (d, *J* = 6.7 Hz, 1.33H), 7.24 (s, 1.33H), 7.16 (d, *J* = 2.6 Hz, 1.33H), 7.04 (t, J = 7.8 Hz, 1.66H), 6.99 (d, *J* = 3.9 Hz, 0.33H), 6.96 (d, *J* = 8.9 Hz, 2.66H), 6.60 (d, J = 8.8 Hz, 1.33H), 4.92 (d, J = 9.8 Hz, 1H), 4.81 (s, 0.33H), 4.64 (s, 1.33H), 4.48 - 4.43 (m, 1.33H), 3.90 (d, J = 1.5 Hz, 3.66H), 3.87 (d, J = 3.5 Hz, 8H), 3.40 - 3.27 (m, 1.66H), 3.22 (d, J = 5.9 Hz, 1H), 3.19 (t, J = 4.9 Hz, 8H), 1.50 (s, 9H), 1.42 (s, 3H).

¹³C-NMR (100 MHz, CDCl₃) δ (ppm): 169.08, 168.77, 155.39, 150.38, 146.88, 146.60, 135.11, 132.97, 131.79, 130.55, 129.77, 129.11, 127.27, 125.69, 124.40, 121.54, 117.80, 117.50, 116.10, 113.52, 112.91, 112.54, 110.10, 110.03, 80.86, 77.36, 67.01, 52.55, 52.03, 49.47, 48.55, 41.16, 28.44, 28.18.

**HPLC** (Sunfire C18, 80-95% of A in B, 5 min): t_{R} = 1.51 min.

**LC-MS (m/z):** 688.26 ([M+H]⁺).

### Example 4:

### Methyl 4-(((1RS,2S)-3-(4-bromo-1H-indol-3-yl)-2-((tert-butoxycarbonyl)amino)-1-cyanopropyl)amino)-4'-morpholino-[1,1'-biphenyl]-3-carboxylate

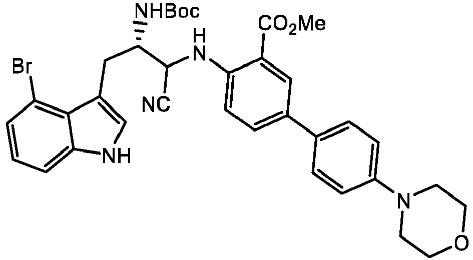

C₃₅H₃₃BrN₅O₅, 687.21 g.mol-1

Obtained following Protocol A and Protocol E.

Purification by flash chromatography, using 0% to 60% of EtOAc in hexane gradient as eluants, furnished the desired compound as an epimeric mixture, ratio 1:3/*1R,2S:1S,2S* in 63 % yield
¹H-NMR (400 MHz, CDCl₃) δ (ppm): 8.38 (d, J = 14.4 Hz, 2.66H), 8.35 - 8.30 (m, 1.33fH), 8.18 - 8.14 (m, 2H), 7.67 (dd, J = 8.7, 2.3 Hz, 0.66H), 7.60 (d, J = 8.9 Hz, 1.66H), 7.48 (t, J = 8.7 Hz, 4.33H), 7.30 (d, J = 7.8 Hz, 3.99H), 7.22 (d, J = 8.4 Hz, 2H), 7.01 (q, J = 8.3 Hz, 6.66H), 6.74 (d, J = 8.8 Hz, 1.66H), 5.02 (d, J = 9.3 Hz, 1.66H), 4.72 (s, 1.33H), 4.65 (s, 1.66H), 4.53 (s, 0.66H), 3.90 (s, 9.33H), 3.87 (s, 4.66H), 3.62 - 3.40 (m, 3.33H), 3.20 (t, J = 4.9 Hz, 8H), 1.41 (s, 9H), 1.35 (s, 3H).

¹³C-NMR (100 MHz, CDCl₃) δ (ppm): 168.93, 168.57, 155.48, 147.13, 146.84, 137.87, 133.22, 132.86, 130.18, 129.78, 129.62, 127.30, 125.45, 125.39, 125.11, 124.52, 124.38, 123.35, 123.28, 117.89, 117.73, 116.39, 114.29, 112.93, 112.59, 111.47, 110.89, 80.43, 66.83, 53.82, 52.09, 51.97, 49.76, 49.04, 29.00, 28.35, 27.67.

**HPLC** (Sunfire C18, 80-95% of A in B, 10 min): t_{R} = 1.36 min.

**LC-MS (m/z):** 688.16 ([M+H]⁺).

### Example 5:

### Methyl 4-(((1RS,2S)-2-((tert-butoxycarbonyl)amino)-1-cyano-3-(5-phenyl-1H-indol-3-yl)propyl)amino)-4'-morpholino-[1,1'-biphenyl]-3-carboxylate

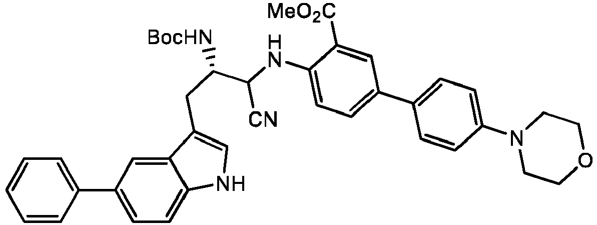

C₄₁H₄₃N₅O₅, 685.83 g.mol-1

Obtained following Protocol A and Protocol D.

Purification by flash chromatography, using 0% to 50% of EtOAc in hexane gradient as eluants, furnished the desired compound as an epimeric mixture, ratio 1:1/*1R,2S:1S,2S* in 30 % yield
¹H-NMR (400 MHz, CDCl₃) δ (ppm): 8.35 (t, *J* = 7.5 Hz, 2H), 8.23 (d, *J* = 2.4 Hz, 2H), 8.22 - 8.18 (m, 2H), 8.15 (d, *J* = 2.3 Hz, 1H), 7.88 (s, 1H), 7.77 (d, *J =* 1.7 Hz, 1H), 7.67 - 7.59 (m, 2H), 7.58 - 7.28 (m, 15H), 7.18 (d, *J* = 2.6 Hz, 2H), 6.96 (dd, *J* = 8.9, 5.7 Hz, 5H), 6.62 (d, *J* = 8.8 Hz, 1H), 4.96 (d, *J* = 9.5 Hz, 1H), 4.89 (s, 1H), 4.79 (s, 1H), 4.67 (s, 1H), 4.56 - 4.47 (m, 2H), 3.90 (d, *J* = 3.1 Hz, 3H), 3.88 (s, 9H), 3.41 (ddd, *J* = 37.0, 14.8, 5.3 Hz, 2H), 3.27 (d, *J* = 7.6 Hz, 2H), 3.24 - 3.13 (m, 8H), 1.48 (s, 9H), 1.40 (s, 9H).

¹³C-NMR (100 MHz, CDCl₃) δ (ppm): 168.89, 168.65, 155.44, 146.86, 142.27, 136.01, 133.89, 133.75, 133.26, 132.96, 129.86, 128.80, 128.73, 127.91, 127.54, 127.45, 126.60, 126.52, 123.98, 122.64, 122.50, 117.85, 117.31, 117.25, 112.85, 112.62, 112.56, 112.45, 111.80, 111.71, 110.50, 80.77, 77.36, 66.38, 52.59, 52.11, 52.03, 48.58, 48.50, 28.43, 28.37.

**HPLC** (Sunfire C18, 70-95% of A in B, 5 min): t_{R} = 3.06 min.

**LC-MS (m/z):** 686.37 ([M+H]⁺).

### Example 6:

### Methyl 4-(((1RS,2S)-2-((tert-butoxycarbonyl)amino)-1-cyano-3-(4-(4-ethynylphenyl)-1H-indol-3-yl)propyl)amino)-4'-morpholino-[1,1'-biphenyl]-3-carboxylate

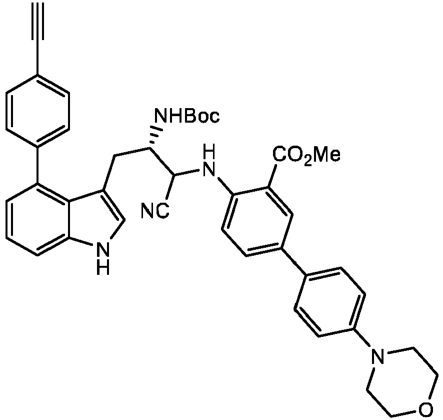

C₄₃H₄₃N₅O₅ 709.33·mol⁻¹

Obtained following Protocol A and Protocol D.

Purification by flash chromatography, using 20% to 40% of AcOEt in hexane gradient as eluants, furnished the desired compound as an epimeric mixture, ratio 1:2/*1R,2S:1S,2S* in 15 % yield
¹H-NMR (500 MHz, CDCl₃) δ (ppm): 8.31 - 8.21 (m, 2H), 8.19 - 8.08 (m, 2H), 8.19 - 8.08 (m, 1H), 7.87 - 7.81 (m, 1H), 7.73 - 7.66 (m, 1H), 7.64 (dd, *J* = 8.6, 2.2 Hz, 1H), 7.61 - 7.47 (m, 7H), 7.61 - 7.47 (m, 5H), 7.46 - 7.37 (m, 3H), 7.46 - 7.37 (m, 3H), 7.25 - 7.24 (m, 1H), 7.23 (s, 1H), 7.23 - 7.21 (m, 1H), 7.15 (s, 1H), 6.97 (dd, *J* = 7.2, 1.0 Hz, 1H), 6.91 (d, *J* = 5.6 Hz, 1H), 6.82 (s, 1H), 6.57 (d, *J* = 8.7 Hz, 1H), 4.47 (d, *J* = 9.4 Hz, 1H), 4.43 - 4.35 (m, 1H), 4.21 - 3.89 (m, 6H), 4.21 - 3.89 (m, 5H), 3.89 (s, 3H), 3.85 (s, 3H), 3.83 - 3.82 (m, 1H), 3.69 - 3.58 (m, 1H), 3.41 - 3.26 (m, 4H), 3.41 - 3.26 (m, 4H), 3.27 - 3.20 (m, 1H), 3.09 (s, 1H), 3.05 (s, 1H), 2.82 (dd, *J* = 14.8, 6.4 Hz, 1H), 2.75 (dd, *J=* 14.6, 8.1 Hz, 1H), 1.40 (s, 9H), 1.36 (s, 9H)

¹³C-NMR (125 MHz, CDCl₃) δ (ppm): 168.5, 168.2, 155.1, 155.1, 142.1, 142.1, 137.4 (C), 137.2, 134.7, 134.7, 133.3, 133.0, 132.0, 132.0, 130.2, 130.2, 129.8, 127.9, 127.9, 127.9, 124.6, 124.1, 122.3, 122.3, 121.9, 121.9, 121.2, 121.2, 117.5, 117.2, 113.0, 113.0, 112.8, 112.8, 111.0, 111.0, 110.9, 110.8, 83.8, 83.8, 80.4, 80.4, 77.4, 77.4, 65.2, 65.2, 52.7, 52.1, 52.0, 52.0, 49.1, 48.7, 30.4, 29.9, 28.4, 28.4.

**HPLC** (Sunfire C18, 60-95% of A in B, 10 min): t_{R} = 6.33 min.

**LC-MS (m/z):** 710.3 ([M+H]⁺).

### Example 7:

### Methyl 2-(((1RS,2S)-2-((tert-butoxycarbonyl)amino)-1-cyano-3-(1H-indol-3-yl)propyl)amino)-5-(2-morpholinopyridin-4-yl)benzoate.

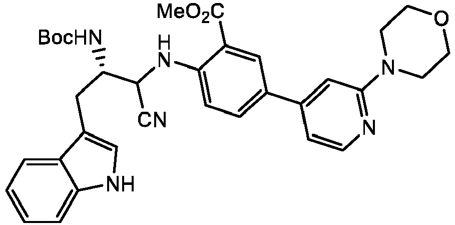

C₃₄H₃₈N₆O₅, 610.72 g·mol⁻¹

Obtained following Protocol A and Protocol D.

Purification by flash chromatography, using 0% to 50% of EtOAc in hexane gradient as eluants, furnished the desired compound as an epimeric mixture, ratio 1:1.5/*1R,2S:1S,2S* in 48 % yield.

¹H-NMR (400 MHz, CDCl₃) δ (ppm): 8.51 (d, *J* = 8.1 Hz, 1H), 8.47 (d, *J* = 8.4 Hz, 0.66H), 8.28 - 8.12 (m, 6H), 7.71 (d, *J* = 11.0 Hz, 1.66H), 7.56 (dd, *J* = 10.5, 6.3 Hz, 2H), 7.40 (t, *J* = 8.6 Hz, 2H), 7.25 - 7.03 (m, 6H), 6.97 (d, *J* = 8.3 Hz, 0.66H), 6.85 (dd, *J* = 14.7, 6.0 Hz, 2.32H), 6.75 (d, *J* = 15.1 Hz, 2.98H), 6.59 (d, *J* = 9.6 Hz, 1H), 4.95 (d, *J* = 10.1 Hz, 1H), 4.85 (s, 1H), 4.68 (s, 1H), 4.52 - 4.42 (m, 2H), 3.92 (t, *J* = 1.8 Hz, 2H), 3.89 (d, *J* = 2.0 Hz, 3H), 3.88 - 3.83 (m, 10H), 3.57 (d, *J* = 4.9 Hz, 10H), 3.44 - 3.29 (m, 2H), 3.21 (dd, *J* = 15.7, 8.0 Hz, 2H), 1.52 (s, 9H), 1.44 (s, 1.66H), 1.42 (d, *J* = 1.8 Hz, 6H).

¹³C-NMR (100 MHz, CDCl₃) δ (ppm): 168.65, 168.36, 160.34, 155.55, 149.15, 148.55, 148.37, 148.18, 136.52, 133.50, 133.12, 132.66, 130.45, 128.29, 127.35, 127.16, 123.16, 122.81, 122.69, 120.23, 120.11, 120.01, 118.93, 118.72, 117.69, 117.37, 112.75, 112.56, 112.06, 111.53, 111.46, 110.14, 109.97, 104.10, 80.89, 77.36, 66.94, 52.53, 52.25, 52.11, 51.90, 48.48, 48.40, 45.97, 28.75, 28.46, 28.37.

**HPLC** (Sunfire C18, 40-95% of A in B, 10 min): t_{R} = 1.61 min.

**LC-MS (m/z):** 611.37 ([M+H]⁺).

### Example 8:

### Methyl 4-(((1RS,2S)-2-((tert-butoxycarbonyl)amino)-1-cyano-3-(1H-indol-3-yl)propyl)amino)-4'-(morpholine-4-carbonyl)-[1,1'-biphenyl]-3-carboxylate.

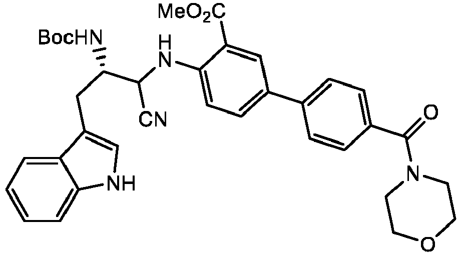

C₃₆H₃₉N₅O₆, 637.74g·mol⁻¹

Obtained following Protocol A and Protocol D.

Purification by flash chromatography, using 40% to 60% of EtOAc in hexane gradient as eluants, furnished the desired compound as an epimeric mixture, ratio 1:1.5/*1R*,*2S*:*1S*,*2S* in 80 % yield
¹H-NMR (400 MHz, CDCl₃) δ (ppm): 8.43 (dd, *J* = 12.5, 8.1 Hz, 1.66H), 8.31 (s, 1H), 8.27 (s, 0.66H), 8.22 (d, *J* = 2.4 Hz, 0.66H), 8.19 (d, *J* = 2.4 Hz, 1H), 7.76 - 7.63 (m, 2.34H), 7.57 (dd, *J* = 14.8, 8.3 Hz, 5H), 7.46 (t, *J* = 8.7 Hz, 4H), 7.38 (t, *J* = 8.2 Hz, 1.66H), 7.32 - 7.07 (m, 7H), 6.95 (d, *J* = 7.8 Hz, 0.66H), 6.83 (d, *J* = 8.7 Hz, 0.66H), 6.59 (d, *J* = 8.9 Hz, 1H), 4.97 (d, *J* = 9.8 Hz, 1H), 4.85 (s, 1H), 4.68 (s, 1H), 4.53 - 4.43 (m, 2H), 3.91 (s, 2H), 3.88 (s, 3H), 3.71 (d, *J* = 11.5 Hz, 20H), 3.44 - 3.29 (m, 2H), 3.20 (dd, *J* = 14.8, 8.7 Hz, 1.66H), 1.51 (s, 9H), 1.42 (s, 7H).

¹³C-NMR (100 MHz, CDCl₃) δ (ppm): 170.50, 168.73, 168.44, 158.17, 155.55, 147.92, 147.53, 141.75, 136.52, 133.55, 133.19, 132.82, 132.66, 132.28, 132.18, 130.43, 129.80, 129.47, 129.25, 128.76, 127.99, 127.95, 127.16, 126.52, 126.32, 123.19, 122.74, 122.63, 120.17, 120.06, 118.92, 118.72, 117.81, 117.42, 115.53, 112.82, 112.65, 112.48, 111.46, 110.09, 109.93, 80.85, 80.67, 77.36, 67.05, 52.54, 52.19, 52.05, 51.84, 48.48, 48.39, 28.45, 28.37.

**HPLC** (Sunfire C18, 60-95% of A in B, 5 min): t_{R} = 1.86 min.

**LC-MS (m/z):** 638.36 ([M+H]⁺).

### Example 9:

### Methyl 2-(((1RS,2S)-2-((tert-butoxycarbonyl)amino)-1-cyano-3-(1H-indol-3-yl)propyl)amino)-5-(1H-indol-5-yl)benzoate

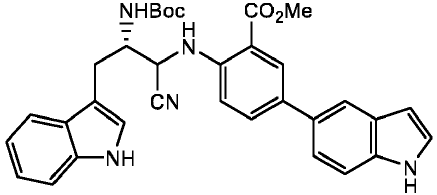

C₃₃H₃₃N₅O₄, 563.66·mol⁻¹

Obtained following Protocol A and Protocol D.

Purification by flash chromatography, using 0% to 50% of EtOAc in hexane gradient as eluants, furnished the desired compound as an epimeric mixture, ratio 1:1.5/*1R*,*2S*:*1S*,*2S* in 70 % yield
¹H-NMR (400 MHz, CDCl₃) δ (ppm): 8.33 (t, *J* = 8.1 Hz, 1.66H), 8.28 - 8.19 (m, 3.66H), 8.15 (d, *J* = 15.3 Hz, 1.66H), 7.82 - 7.75 (m, 1.66H), 7.75 - 7.70 (m, 1.66H), 7.65 - 7.58 (m, 1.66H), 7.47 - 7.33 (m, 5.66H), 7.25 - 7.19 (m, 3.66H), 7.16 (s, 2H), 7.13 (d, *J* = 7.9 Hz, 0.66H), 7.10 (d, *J* = 6.4 Hz, 0.66H), 6.91 (d, *J* = 7.9 Hz, 0.66H), 6.63 - 6.56 (m, 2.66H), 4.95 (d, *J* = 9.6 Hz, 1H), 4.84 (s, 1H), 4.68 (s, 1H), 4.56 - 4.45 (m, 2H), 3.92 (s, 2H), 3.89 (s, 3H), 3.37 (ddd, *J* = 26.4, 15.6, 6.5 Hz, 1.66H), 3.22 (dd, *J* = 14.7, 8.5 Hz, 2H), 1.52 (s, 9H), 1.43 (s, 7H).

¹³C-NMR (100 MHz, CDCl₃) δ(ppm): 169.13, 168.82, 155.55, 146.88, 146.49, 136.48, 135.21, 134.04, 133.66, 132.32, 132.16, 130.44, 128.57, 127.41, 125.04, 123.15, 122.73, 122.63, 121.44, 120.19, 120.09, 118.98, 118.82, 118.56, 118.08, 117.70, 112.80, 112.58, 112.49, 111.48, 111.43, 111.39, 110.24, 110.13, 103.01, 80.76, 80.59, 77.36, 52.65, 52.08, 51.93, 48.59, 48.48, 28.56, 28.47, 28.39.

**HPLC** (Sunfire C18, 60-95% of A in B, 5 min): t_{R} = 3.83 min.

**LC-MS (m/z):** 564.15 ([M+H]⁺).

### Example 10:

### Methyl 4-(((1RS,2S)-2-((tert-butoxycarbonyl)amino)-1-cyano-3-(1H-indol-3-yl)propyl)amino)-4'-(morpholinomethyl)-[1,1'-biphenyl]-3-carboxylate

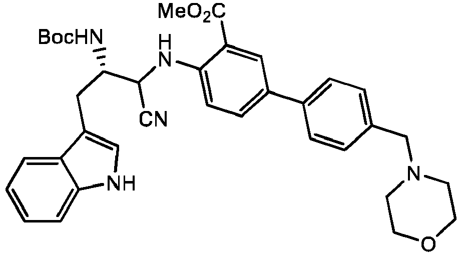

C₃₆H₄₁N₅O₅, 623.75·mol⁻¹

Obtained following Protocol A and Protocol D.

Purification by flash chromatography, using 20% to 80% of EtOAc in hexane gradient as eluants, furnished the desired compound as an epimeric mixture, ratio 1:1.5/*1R*,*2S*:*1S*,*2S* in 65 % yield
¹H-NMR (400 MHz, CDCl₃) δ (ppm): 8.37 (t, *J* = 8.7 Hz, 1.66H), 8.20 (dd, *J* = 12.3, 2.3 Hz, 3.32H), 7.74 - 7.65 (m, 2H), 7.62 - 7.54 (m, 2H), 7.49 (dd, *J* = 14.2, 8.3 Hz, 4H), 7.38 (q, *J* = 7.8 Hz, 6H), 7.21 (t, *J* = 7.9 Hz, 2H), 7.19 - 7.12 (m, 3.32H), 7.10 (d, *J* = 4.3 Hz, 1.32H), 6.93 (d, *J* = 8.2 Hz, 0.66H), 6.76 (d, *J* = 8.6 Hz, 0.66H), 6.59 (d, *J* = 8.8 Hz, 1H), 4.94 (d, *J* = 9.8 Hz, 1H), 4.83 (s, 1H), 4.67 (s, 1H), 4.53 - 4.43 (m, 2H), 3.91 (s, 2H), 3.88 (s, 3H), 3.72 (dq, *J* = 9.3, 4.3 Hz, 7.66H), 3.53 (d, *J* = 5.9 Hz, 4H), 3.42 (s, 1H), 3.41 - 3.29 (m, 2H), 3.21 (dd, *J* = 14.8, 8.4 Hz, 2H), 2.47 (d, *J* = 4.6 Hz, 8H), 2.43 (d, *J* = 4.8 Hz, 1.66H), 1.51 (s, 9H), 1.42 (s, 7H).

¹³C-NMR (100 MHz, CDCl₃)δ (ppm): 168.90, 168.61, 155.54, 155.16, 147.49, 147.10, 139.04, 136.53, 133.60, 133.22, 130.74, 130.36, 129.86, 126.38, 123.14, 122.76, 122.66, 120.21, 120.10, 118.78, 117.91, 117.53, 115.27, 112.81, 112.54, 111.49, 110.24, 110.09, 80.80, 77.36, 67.17, 63.25, 62.99, 53.77, 53.63, 52.12, 51.77, 48.57, 48.45, 28.66, 28.47, 28.38.

**HPLC** (Sunfire C18, 30-95% of A in B, 5 min): t_{R} = 3.96 min.

**LC-MS (m/z):** 624.41 ([M+H]⁺).

### Example 11:

### Methyl 2-(((1RS,2S)-2-((tert-butoxycarbonyl)amino)-1-cyano-3-(1H-indol-3-yl)propyl)amino)-5-(1H-indol-6-yl)benzoate

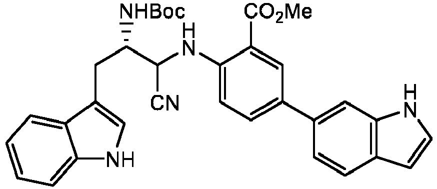

C₃₃H₃₃N₅O₄, 563.66·mol⁻¹

Obtained following Protocol A and Protocol D.

Purification by flash chromatography, using 0% to 40% of EtOAc in hexane gradient as eluants, furnished the desired compound as an epimeric mixture, ratio 1:1.5/*1R,2S:1S,2S* in 80 % yield
¹H-NMR (400 MHz, CDCl₃) δ (ppm): 8.34 (t, *J* = 7.9 Hz, 1.66H), 8.28 - 8.11 (m, 5.32H), 7.74 - 7.64 (m, 3.66H), 7.59 (t, *J* = 8.1 Hz, 2H), 7.55 (s, 0.66H), 7.50 (s, 1H), 7.39 (t, *J* = 7.9 Hz, 1.66H), 7.34 (dd, J = 8.3, 1.5 Hz, 1H), 7.29 (d, *J* = 6.6 Hz, 1.32H), 7.26 - 7.19 (m, 4H), 7.18 - 7.07 (m, 4H), 6.91 (d, *J* = 8.1 Hz, 0.66H), 6.57 (dd, *J* = 6.0, 3.0 Hz, 3H), 4.96 (d, *J* = 9.9 Hz, 1H), 4.84 (s, 1H), 4.68 (s, 1H), 4.54 - 4.42 (m, 2H), 3.91 (s, 2H), 3.85 (s, 3H), 3.43 - 3.29 (m, 1.66H), 3.21 (dd, *J* = 14.7, 8.5 Hz, 2H), 1.52 (s, 9H), 1.42 (s, 7H).

¹³C-NMR (100 MHz, CDCl₃) δ (ppm): 169.05, 168.73, 155.58, 147.06, 146.63, 136.57, 136.48, 134.44, 133.96, 133.53, 131.83, 131.73, 130.43, 127.40, 127.22, 127.05, 127.00, 124.82, 123.15, 122.76, 122.64, 121.09, 121.04, 120.21, 120.09, 119.26, 118.95, 118.81, 118.03, 117.66, 112.79, 112.60, 112.50, 111.51, 111.43, 110.22, 110.10, 108.90, 102.68, 102.63, 80.81, 77.36, 52.66, 52.10, 51.93, 48.57, 48.49, 28.63, 28.48, 28.39.

**HPLC** (Sunfire C18, 60-95% of A in B, 5 min): t_{R} = 4.21 min.

**LC-MS (m/z):** 564.15 ([M+H]⁺).

### Example 12:

### Methyl 5-(benzofuran-2-yl)-2-(((1RS,2S)-2-((tert-butoxycarbonyl)amino)-1-cyano-3-(1H-indol-3-yl)propyl)amino)benzoate

C₃₃H₃₂N₄O₅, 564.24·mol⁻¹

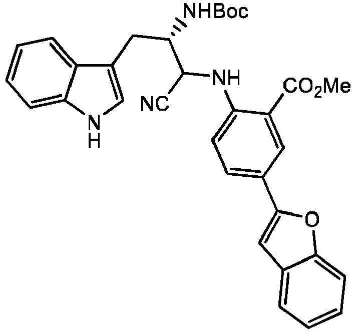

Obtained following Protocol A and Protocol D.

Purification by flash chromatography, using 0% to 50% of EtOAc in hexane gradient as eluants, furnished the desired compound as an epimeric mixture, ratio 1:1.5/*1R,2S:1S,2S* in 82 % yield
¹H-NMR (400 MHz, CDCl₃) δ (ppm): 8.54 (d, J = 8.3 Hz, 1H), 8.50 (d, J = 7.9 Hz, 0.66H), 8.47 (d, J = 2.2 Hz, 0.66H), 8.44 (s, 1H), 8.20 (s, 1H), 8.15 (s, 0.66H), 7.90 (dd, J = 8.7, 2.3 Hz, 0.66H), 7.79 (d, J = 6.5 Hz, 1H), 7.72 (d, J = 7.2 Hz, 1H), 7.60 - 7.47 (m, 5H), 7.43 - 7.36 (t, 2H), 7.25 - 7.16 (m, 7H), 7.14 (d, J = 5.3 Hz, 0.66H), 7.12 - 7.09 (m, 1.22H), 6.88 (d, J = 13.0 Hz, 1.66H), 6.58 (d, J = 8.8 Hz, 1H), 4.95 (d, J = 10.4 Hz, 1H), 4.85 (s, 1H), 4.69 (s, 1.66H), 4.54 - 4.44 (s, 2H), 3.94 (s, 3H), 3.92 (s, 4H), 3.34 (dd, J = 21.7, 5.8 Hz, 2H), 3.21 (dd, J = 14.7, 8.6 Hz, 2H), 1.52 (s, 9H), 1.43 (s, 6H).

¹³C-NMR (100 MHz, CDCl₃) δ (ppm): 168.65, 168.36, 155.53, 155.42, 154.81, 154.78, 148.23, 147.86, 136.53, 131.65, 131.26, 129.55, 128.63, 128.58, 127.37, 127.18, 123.99, 123.95, 123.15, 123.07, 123.03, 122.81, 122.70, 120.73, 120.69, 120.36, 120.30, 120.24, 120.13, 118.93, 118.73, 117.69, 117.30, 112.63, 112.47, 112.39, 112.26, 111.53, 111.45, 111.14, 111.11, 110.16, 109.99, 99.79, 99.73, 80.88, 52.60, 52.24, 52.11, 48.44, 48.35, 28.71, 28.46, 28.37.

**HPLC** (Sunfire C18, 80-95% of A in B, 5 min): t_{R} = 1.89 min.

**LC-MS (m/z):** 587.44 ([M+Na]⁺).

### Example 13:

### Methyl 5-(benzo[b]thiophen-2-yl)-2-(((1RS,2S)-2-((tert-butoxycarbonyl)amino)-1-cyano-3-(1H-indol-3-yl)propyl)amino)benzoate

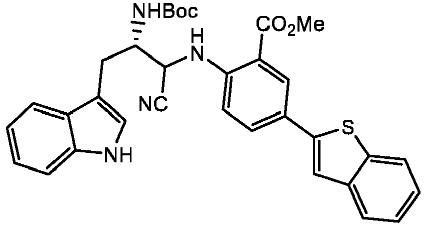

C₃₃H₃₂N₄O₄S 580.21·mol⁻¹

Obtained following Protocol A and Protocol D.

Purification by flash chromatography, using 0% to 50% of EtOAc in hexane gradient as eluants, furnished the desired compound as an epimeric mixture, ratio 1:1.5/*1R,2S:1S,2S* in 82 % yield
¹H-NMR (400 MHz, CDCl₃) δ (ppm): 8.46 (d, J = 8.3 Hz, 1H), 8.42 (d, *J* = 9.4 Hz, 1H)), 8.20 (d, J = 2.2 Hz, 0.66H), 8.19 - 8.14 (m, 3H), 7.87 - 7.84 (m, 0.8H) 7.93 - 7.89 (m, 1.2H), 7.83 - 7.80 (m, 1H), 7.73 (d, J = 8.2 Hz, 1H), 7.66 (dd, J = 8.6, 2.3 Hz, 1H), 7.61 (d, J = 7.9 Hz, 1H), 7.55 (s, 1.22H), 7.42 - 7.37 (m, 6H), 7.35 (s, 0.66H), 7.31 (s, 1H), 7.23 (dd, J = 8.3, 1.2 Hz, 1.66H), 7.19 (m, 1.8H), 7.16 (s, 0.66H), 7.15 (s, 0.66H), 7.13 (s, 1H), 6.98 (d, J = 8.1 Hz, 0.66H), 6.63 (d, J = 8.6 Hz, 1H), 4.96 (d, J = 9.7 Hz, 1H), 4.86 (s, 1.4H), 4.69 (d, J = 4.8 Hz, 1.4H), 4.56 - 4.47 (m, 2.33H), 3.90 (s, 2H), 3.87 (s, 3H), 3.38 (ddd, J = 28.6, 15.0, 5.6 Hz, 2.2H), 3.23 (dd, J = 14.7, 8.6 Hz, 2.2H), 1.53 (s, 9H), 1.43 (s, 6H).

¹³C-NMR (100 MHz, CDCl₃) δ (ppm): 168.82, 168.53, 155.55, 147.65, 147.26, 140.74, 138.07, 137.02, 136.53, 135.33, 134.96, 132.07, 127.41, 127.21, 125.48, 125.39, 124.55, 124.52, 124.48, 123.16, 123.12, 123.09, 122.92, 122.89, 122.84, 122.77, 122.66, 120.21, 120.10, 118.96, 118.77, 117.91, 117.52, 112.71, 112.46, 112.37, 111.51, 111.45, 110.22, 110.07, 80.83, 80.66, 77.36, 52.65, 52.16, 52.02, 48.56, 48.43, 28.69, 28.47, 28.39, 26.30.

**HPLC** (Sunfire C18, 80-95% of A in B, 5 min): t_{R} = 2.04 min.

**LC-MS (m/z):** 603.38 ([M+Na]⁺).

### Example 14:

### tert-Butyl 4-(4-(((1RS,2S)-2-((tert-butoxycarbonyl)amino)-1-cyano-3-(1H-indol-3-yl)propyl)amino)-3-(methoxycarbonyl)phenyl)-3,6-dihydropyridine-1(2H)-carboxylate

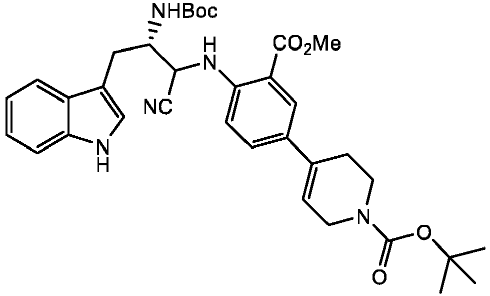

C₃₅H₄₃N₅O₆ 629.32·mol⁻¹

Obtained following Protocol A and Protocol D.

Purification by flash chromatography, using 0% to 50% of EtOAc in hexane gradient as eluants, furnished the desired compound as an epimeric mixture, ratio 1:1.5/*1R,2S:1S,2S* in 75 % yield
¹H-NMR (400 MHz, CDCl₃) δ (ppm): 8.32 (t, J = 7.9 Hz, 1.66H), 8.17 (s, 1H), 8.14 (s, 0.66H), 7.97 (d, J = 2.3 Hz, 0.66H), 7.94 (d, J = 2.4 Hz, 1H), 7.70 (d, J = 7.6 Hz, 1H), 7.57 (d, J = 7.9 Hz, 1H), 7.48 (dd, J = 8.8, 2.3 Hz, 0.66H), 7.42 - 7.34 (m, 3.4H), 7.25 - 7.19 (m, 2.32H), 7.17 (s, 1.66H), 7.12 (dd, J = 16.1, 8.1 Hz, 2.66H), 6.84 (s, 0.66H), 6.49 (d, J = 8.9 Hz, 1H), 5.92 (s, 1H), 4.91 (d, J = 9.7 Hz, 1H), 4.80 (s, 1.22H), 4.65 (s, 1.22H), 4.49 - 4.39 (m, 2H), 4.06 (d, J = 6.1 Hz, 3H), 3.89 (s, 2H), 3.86 (s, 3H), 3.66 - 3.59 (m, 4H), 3.40 - 3.27 (m, 2.33H), 3.19 (dd, J = 14.7, 8.5 Hz, 2.33H), 2.46 (s, 4H), 1.58 (s, 5H), 1.49 (s, 9H), 1.48 (s, 9H), 1.41 (s, 6H).

¹³C-NMR (100 MHz, CDCl₃) δ (ppm): 167.70, 167.40, 154.34, 153.84, 146.23, 145.84, 135.35, 130.51, 130.12, 128.96, 127.02, 126.97, 126.24, 126.05, 121.97, 121.60, 121.49, 119.03, 118.93, 117.80, 117.61, 116.72, 116.33, 111.15, 111.05, 110.98, 110.32, 110.26, 109.08, 108.93, 79.61, 78.66, 51.41, 50.92, 50.78, 47.39, 47.27, 27.47, 27.30, 27.21, 26.18.

**HPLC** (Sunfire C18, 70-95% of A in B, 5 min): t_{R} = 3.41 min.

**LC-MS (m/z):** 630.15 ([M+H]⁺).

### Example 15:

### Methyl 5-(1H-benzo[d]imidazol-5-yl)-2-(((1RS,2S)-2-((tert-butoxycarbonyl)amino)-1-cyano-3-(1H-indol-3-yl)propyl)amino)benzoate

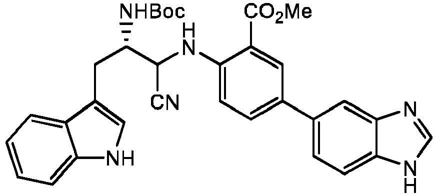

C₃₂H₃₂N₆O₄ 564.6·mol⁻¹

Obtained following Protocol B and Protocol D.

Purification by flash chromatography, using 0% to 2% of MeOH in AcOEt gradient as eluants, furnished the desired compound as an epimeric mixture, ratio 1:2/*1R,2S:1S,2S* in 38 % yield
¹H-NMR (400 MHz, CDCl₃) δ (ppm): 8.49 (s, 1H), 8.34 (d, J = 8.4 Hz, 1H), 8.31 (d, J = 7.9 Hz, 0.5H), 8.17 (s, 0.5H), 8.12 (d, J = 6.4 Hz, 3H), 7.72 - 7.54 (m, 6H), 7.39 (q, J = 8.8 Hz, 4.5H), 7.22 - 7.07 (m, 5.5H), 6.82 (s, 0.5H), 6.47 (d, J = 8.7 Hz, 1H), 5.04 (d, J = 9.8 Hz, 1H), 4.96 (m, 0.5H), 4.76 (m, 0.5H), 4.67 (m, 1H), 4.51 (p, J = 7.8 Hz, 0.5H), 4.44 (d, J = 9.1 Hz, 1H), 3.85 (s, 1.5H), 3.80 (s, 3H), 3.40 - 3.28 (m, 1.5H), 3.20 (dd, J = 14.7, 8.5 Hz, 1.5H), 1.52 (s, 9H), 1.41 (s, 4.5H).

¹³C-NMR (100 MHz, CDCl₃) δ (ppm): 167.70, 167.40, 154.55, 146.13, 145.65, 139.77, 135.41, 134.68, 132.30, 129.46, 126.04, 122.12, 121.57, 121.44, 121.31, 118.99, 118.89, 117.68, 117.58, 116.43, 114.95, 111.59, 110.48, 110.36, 79.78, 51.55, 50.95, 50.78, 47.34, 39.97, 27.33, 27.23.

**HPLC** (Sunfire C18, 30-95% of A in B, 5 min): t_{R} = 4.01 min.

**LC-MS (m/z):** 565 ([M+H]⁺).

### Example 16:

### Methyl 2-(((1RS,2S)-2-((tert-butoxycarbonyl)amino)-1-cyano-3-(1H-indol-3-yl)propyl)amino)-5-(1H-indazol-5-yl)benzoate

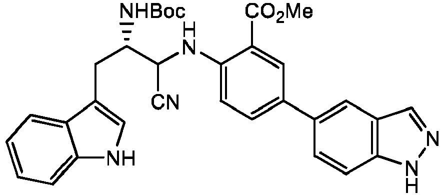

C₃₂H₃₂N₆O₄ 564.6·mol⁻¹

Obtained following Protocol B and Protocol D.

Purification by flash chromatography, using 0% to 60% of AcOEt in hexane gradient as eluants, furnished the desired compound as an epimeric mixture, ratio 1:2/*1R*,*2S*:*1S*,*2S* in 44 % yield.

¹H-NMR (400 MHz, CDCl₃) δ (ppm): 8.30 (t, J = 9.0 Hz, 1.5H), 8.17 (d, J = 17.5 Hz, 1H), 8.06 (dd, J = 12.4, 2.2 Hz, 1.5H), 7.80 - 7.63 (m, 4H), 7.61 - 7.51 (m, 1H), 7.44 (d, J = 8.6 Hz, 1H), 7.39 (tt, J = 8.2, 1.0 Hz, 1H), 7.22 (qd, J = 8.2, 1.5 Hz, 2H), 7.18 - 7.08 (m, 2H), 6.53 (d, J = 8.6 Hz, 1H), 4.94 (m, 1.5H), 4.82 (m, 1H), 4.65 (s, 1.5H), 4.47 (m, 1.5H), 3.90 (s, 1.5H), 3.87 (s, 3H), 3.42 - 3.27 (m, 1H), 3.20 (dd, J = 14.7, 8.5 Hz, 1H), 1.50 (s, 9H), 1.41 (s, 4.5H)

¹³C-NMR (100 MHz, CDCl₃) δ (ppm): 168.38, 167.74, 155.47, 146.58, 136.93, 136.44, 132.13, 128.95, 127.16, 123.26, 123.10, 122.70, 122.59, 120.14, 120.04, 118.90, 112.74, 112.57, 111.44, 110.15, 80.75, 53.27, 52.49, 52.09, 51.95, 48.48, 28.61, 28.41.

**HPLC** (Sunfire C18, 60-95% of A in B, 5 min): t_{R} = 1.86 min.

**LC-MS (m/z):** 565 ([M+H]⁺).

### Example 17:

### Methyl 5-(1-benzyl-1H-pyrazol-4-yl)-2-(((1RS,2S)-2-((tert-butoxycarbonyl)amino)-1-cyano-3-(1H-indol-3-yl)propyl)amino)benzoate

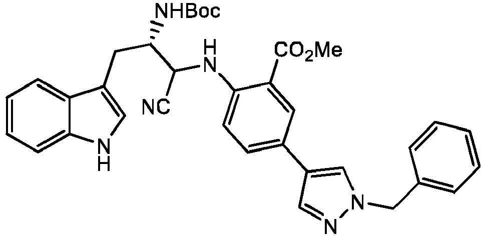

C₃₆H₃₆N₆O₄ 604.7·mol⁻¹

Obtained following Protocol B and Protocol D.

Purification by flash chromatography, using 0% to 60% of AcOEt in hexane gradient as eluants, furnished the desired compound as an epimeric mixture, ratio 1:2/*1R*,*2S*:*1S*,*2S* in 30 % yield.

¹H-NMR (400 MHz, CDCl₃) δ (ppm): 8.28 (t, J = 8.8 Hz, 1.5H), 8.19 (s, 1H), 8.15 (s, 0.5H), 8.04 (d, J = 2.2 Hz, 0.5H), 8.01 (d, J = 2.2 Hz, 1H), 7.78 (d, J = 0.8 Hz, 0.5H), 7.75 - 7.73 (m, 1H), 7.70 (d, J = 7.9 Hz, 1H), 7.60 - 7.49 (m, 3.5H), 7.43 - 7.31 (m, 9H), 7.28 (dd, J = 7.1, 1.8 Hz, 2.5H), 7.25 (t, J = 1.3 Hz, 1H), 7.24 - 7.18 (m, 2H), 7.16 (d, J = 2.2 Hz, 1H), 7.15 (s, 0.5H), 7.12 (dd, J = 2.3, 1.0 Hz, 0.5H), 7.09 (dd, J = 7.6, 1.5 Hz, 1H), 6.85 (s, 0.5H), 6.51 (d, J = 8.7 Hz, 1H), 5.35 (s, 1H), 5.34 (s, 2H), 4.92 (d, J = 9.7 Hz, 1H), 4.80 (s, 1H), 4.65 (s, 1.5H), 4.51 - 4.37 (m, 2H), 3.89 (s, 1.5H), 3.86 (s, 3H), 3.34 (ddd, J = 22.6, 14.8, 5.7 Hz, 2H), 3.19 (dd, J = 14.7, 8.6 Hz, 2H), 1.50 (s, 9H), 1.41 (s, 4.5H).

¹³C-NMR (100 MHz, CDCl₃) δ (ppm): 168.67, 168.37, 155.47, 146.87, 146.49, 136.44, 136.13, 132.42, 132.02, 129.03, 128.76, 128.38, 127.90, 127.14, 125.90, 123.09, 122.85, 122.70, 122.59, 120.14, 120.03, 118.89, 118.71, 117.44, 112.76, 112.59, 111.44, 110.14, 80.75, 56.25, 52.49, 52.08, 51.95, 48.50, 48.42, 28.63, 28.41.

**HPLC** (Sunfire C18, 60-95% of A in B, 5 min): t_{R} = 3.59 min.

**LC-MS (m/z):** 605 ([M+H]⁺).

### Example 18:

### Methyl 2-(((1RS,2S)-2-((tert-butoxycarbonyl)amino)-1-cyano-3-(1H-indol-3-yl)propyl)amino)-5-(1H-pyrrolo[2,3-b]pyridin-3-yl)benzoate

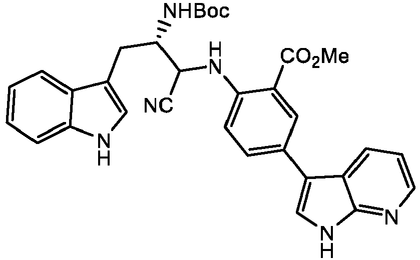

C₃₂H₃₂N₆O₄ 564.6·mol⁻¹

Obtained following Protocol B and Protocol D.

Purification by flash chromatography, using 0% to 70% of AcOEt in hexane gradient as eluants, furnished the desired compound as an epimeric mixture, ratio 1:2/*1R*,*2S*:*1S*,*2S* in 43 % yield.

¹H-NMR (400 MHz, CDCl₃) δ (ppm): 9.55 (s, 1H), 8.48 - 8.32 (m, 3H), 8.27 (s, 0.5H), 8.20 (d, J = 10.3 Hz, 1.5H), 8.06 (d, J = 12.2 Hz, 1.5H), 7.75 - 7.64 (m, 2H), 7.64 - 7.52 (m, 1H), 7.43 - 7.32 (m, 3.5H), 7.25 - 7.08 (m, 4.5H), 6.95 (s, 0.5H), 6.61 (d, J = 8.7 Hz, 1H), 6.53 (s, 1H), 5.20 (s, 1.5H), 5.07 - 4.98 (m, 1H), 4.68 (s, 1H), 4.55 - 4.45 (m, 1.5H), 3.91 (s, 1.5H), 3.89 (s, 3H), 3.45 - 3.29 (m, 1H), 3.22 (dd, J = 14.8, 8.5 Hz, 1.5H), 1.51 (s, 9H), 1.42 (s, 4.5H).

¹³C-NMR (100 MHz, CDCl₃) δ (ppm): 168.66, 168.38, 155.46, 147.34, 146.96, 140.74, 136.42, 133.41, 132.06, 130.44, 128.91, 127.43, 127.10, 126.07, 123.08, 122.63, 122.52, 120.07, 119.96, 118.83, 118.65, 112.86, 112.67, 111.41, 111.34, 110.00, 101.36, 80.71, 52.48, 52.05, 51.92, 48.43, 48.35, 28.39.

**HPLC** (Sunfire C18, 60-95% of A in B, 5 min): t_{R} = 1.62 min.

**LC-MS (m/z):** 565 ([M+H]⁺).

### Example 19:

### tert-Butyl 5-(4-(((1RS,2S)-2-((tert-butoxycarbonyl)amino)-1-cyano-3-(1H-indol-3-yl)propyl)amino)-3-(methoxycarbonyl)phenyl)-1H-indole-1-carboxylate

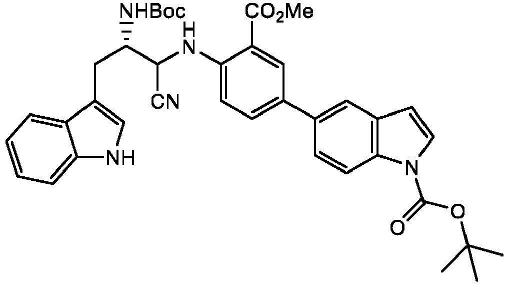

C₃₈H₄₁N₅O₆ 663.8·mol⁻¹

Obtained following Protocol A and Protocol D.

Purification by flash chromatography, using 0% to 40% of AcOEt in hexane gradient as eluants, furnished the desired compound as an epimeric mixture, ratio 1:2/*1R*,*2S*:*1S*,*2S* in 47 % yield.

¹H-NMR (400 MHz, CDCl₃) δ (ppm): 8.36 (t, J = 8.6 Hz, 1.5H), 8.26 (d, J = 2.3 Hz, 0.5H), 8.23 (d, J = 2.3 Hz, 1H), 8.16 (s, 1.5H), 8.13 (s, 1H), 7.74 (d, J = 8.6 Hz, 1H), 7.70 (dd, J = 14.2, 1.8 Hz, 2H), 7.65 - 7.58 (m, 3H), 7.51 (dd, J = 8.7, 1.8 Hz, 0.5H), 7.47 (dd, J = 8.6, 1.9 Hz, 1H), 7.40 (t, J = 8.1 Hz, 1.5H), 7.25 - 7.21 (m, 1H), 7.19 (d, J = 3.0 Hz, 1.5H), 7.15 (d, J = 8.4 Hz, 1H), 7.12 - 7.10 (m, 1H), 6.60 (dd, J = 7.0, 3.8 Hz, 2.5H), 4.94 (d, J = 9.8 Hz, 1H), 4.84 (s, 1H), 4.68 (s, 1H), 4.50 (ddd, J = 15.0, 9.8, 5.9 Hz, 1.5H), 3.92 (d, J = 1.2 Hz, 1.5H), 3.89 (s, 3H), 3.87 - 3.80 (m, 0.5H), 3.63 (m, 0.5H), 3.37 (ddd, J = 25.9, 15.2, 5.8 Hz, 1.5H), 3.22 (dd, J = 14.6, 8.5 Hz, 1.5H), 1.69 (d, J = 3.0 Hz, 13H), 1.51 (s, 9H), 1.42 (s, 4.5H).

13C-NMR (100 MHz, CDCl₃) δ (ppm): 168.95, 168.65, 155.48, 149.82, 147.18, 146.79, 136.45, 134.88, 134.36, 133.88, 133.50, 131.23, 131.04, 130.40, 127.18, 126.59, 123.17, 123.09, 122.72, 122.61, 120.16, 120.06, 118.94, 118.76, 118.66, 117.92, 117.54, 115.47, 115.44, 112.77, 112.59, 112.50, 111.39, 110.21, 110.08, 107.53, 104.30, 83.86, 80.73, 52.56, 52.08, 51.94, 48.52, 48.42, 31.69, 28.58, 28.42, 28.31.

**HPLC** (Sunfire C18, 80-95% of A in B, 5 min): t_{R} = 3.08 min.

**LC-MS (m/z):** 665 ([M+H]⁺).

### Example 20:

### Methyl 4-(((1RS,2S)-2-((tert-butoxycarbonyl)amino)-1-cyano-3-(4-(4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl)-1H-indol-3-yl)propyl)amino)-4'-morpholino-[1,1'-biphenyl]-3-carboxylate

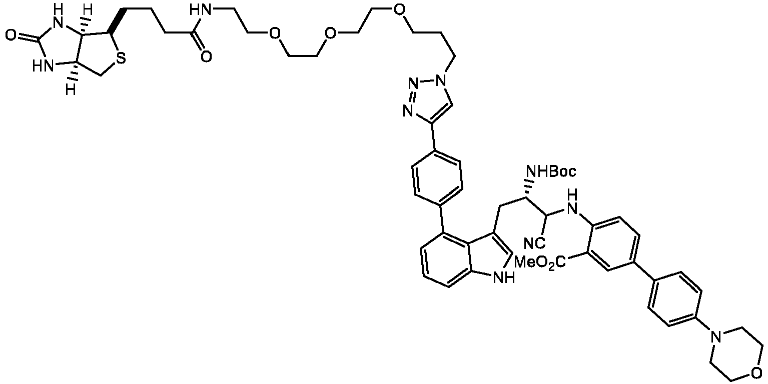

C₆₁H₇₅N₁₁O₁₀S 1153.54·mol⁻¹

Obtained from the compound of example 6 following Protocol C.

Purification by flash chromatography, using 0% to 10% of MeOH in DCM gradient as eluants, furnished the desired compound as an epimeric mixture, ratio 1:1.5/*1R,2S:1S,2S* in 53 % yield
¹H-NMR (400 MHz, CDCl₃) δ (ppm): 8.66 (s, 1H), 8.55 (s, 1H), 8.09 (d, J = 2.2 Hz, 2H), 8.02 (d, J = 12.6 Hz, 2H), 7.92 (s, 4H), 7.86 (s, 1H), 7.83 (d, J = 8.4 Hz, 1H), 7.56 - 7.51 (m, 4H), 7.46 (d, J = 8.8 Hz, 6H), 7.42 (td, J = 8.4, 0.9 Hz, 4H), 7.37 (s, 1H), 7.25 - 7.21 (m, 2H), 7.20 (d, J = 2.1 Hz, 1H), 7.14 (s, 1H), 7.02 - 6.94 (m, 7H), 6.48 (s, 2H), 6.40 (d, J = 8.6 Hz, 1H), 5.79 (s, 1H), 5.72 (s, 1H), 5.01 (s, 1H), 4.99 (s, 1H), 4.73 (d, J = 9.5 Hz, 1H), 4.52 - 4.41 (m, 6H), 4.37 (d, J = 7.1 Hz, 1H), 4.33 (d, J = 12.5 Hz, 2H), 4.27 - 4.22 (m, 3H), 3.91 - 3.86 (m, 13H), 3.84 (s, 1H), 3.81 (s, 4H), 3.80 - 3.76 (m, 3H), 3.69 - 3.62 (m, 6H), 3.58 - 3.53 (m, 11H), 3.53 (s, 6H), 3.51 - 3.35 (m, 13H), 3.20 (t, J = 4.8 Hz, 8H), 3.16 - 3.05 (m, 4H), 2.93 - 2.81 (m, 5H), 2.74 - 2.65 (m, 4H), 2.24 - 2.19 (m, 1H), 2.09 (dt, J = 22.1, 6.2 Hz, 5H), 1.68 (s, 27H), 1.31 (s, 9H), 1.28 (s, 6H), 1.25 (s, 8H).

¹³C-NMR (100 MHz, CDCl₃) δ (ppm): 173.34, 168.68, 163.71, 155.45, 151.18, 148.52, 147.45, 147.0, 139.56, 137.29, 135.06, 133.2, 132.9, 132.8, 132.45, 130.40, 129.31, 128.9, 128.8, 128.1, 128.0, 127.6, 127.4, 127.15, 126.79, 126.2, 126.1, 125.58, 124.75, 124.27, 122.11, 121.29, 118.0, 117.40, 116.55, 114.22, 112.74, 112.4, 110.98, 110.5, 80.17, 70.64, 70.49, 70.20, 69.93, 69.60, 69.45, 66.81, 64.16, 61.96, 60.26, 55.56, 52.84, 51.89, 50.82, 50.32, 49.65, 47.26, 40.64, 39.26, 35.91, 29.84, 28.29, 27.58, 25.58.

**HPLC** (Sunfire C18, 50-95% of A in B, 5 min): t_{R} = 2.1 min.

**LC-MS (m/z):** 1154.60 ([M+H]⁺).

### Example 21:

### Methyl 4-(((1RS,2S)-2-((tert-butoxycarbonyl)amino)-1-cyano-3-(1H-indol-3-yl)propyl)amino)-4'-fluoro-3'-(2H-tetrazol-5-yl)-[1,1'-biphenyl]-3-carboxylate

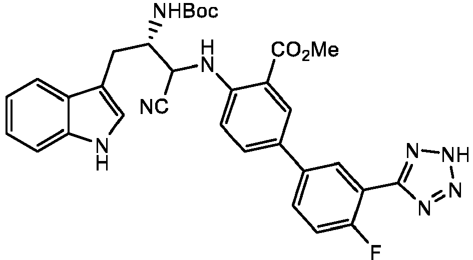

C₃₂H₃₁FN₈O₄ 610.25·mol⁻¹

Obtained following Protocol A and Protocol D.

Purification by flash chromatography, using 3% to 10% of MeOH in DCM gradient as eluants, furnished the desired compound as an epimeric mixture, ratio 1:1.3/*1R,2S:1S,2S* in 54 % yield
¹H-NMR (400 MHz, CDCl₃) δ 8.44 (d, J = 9.4 Hz, 2.5H), 8.39 (d, J = 7.8 Hz, 1.54H), 8.27 (s, 0.77H), 8.14 (s, 0.77H), 8.11 (d, J = 3.8 Hz, 1H), 7.86 (s, 1H), 7.79 (d, J = 7.8 Hz, 1H), 7.61 (d, J = 7.8 Hz, 1.77H), 7.53 (d, J = 7.2 Hz, 1H), 7.46 (d, J = 7.9 Hz, 1.54H), 7.40 (d, J = 7.9 Hz, 2.3H), 7.34 (s, 1H), 7.26 (d, J = 19.8 Hz, 3.77H), 7.20 (t, J = 7.0 Hz, 3.77H), 7.14 (d, J = 2.6 Hz, 1H), 7.11 (d, J = 7.3 Hz, 0.77H), 6.95 (t, J = 9.5 Hz, 1H), 6.87 (s, 0.77H), 6.20 (d, J = 8.9 Hz, 1H), 5.09 (d, J = 10.0 Hz, 1H), 4.92 (d, J = 7.7 Hz, 0.77H), 4.79 (s, 1.77H), 4.56 - 4.47 (m, 1H), 4.38 (dd, J = 8.8, 3.8 Hz, 1H), 3.90 (s, 2.3H), 3.79 (s, 3H), 3.39 (dt, J = 13.9, 6.8 Hz, 2.3H), 3.21 (dd, J = 14.5, 9.7 Hz, 2.3H), 1.56 (s, 9H), 1.43 (s, 6.97H).

¹³C-NMR (100 MHz, CDCl₃) δ (ppm): 168.54, 168.04, 160.17, 159.89, 157.69, 157.41, 156.15, 155.79, 147.90, 147.13, 136.97, 136.57, 133.19, 132.49, 131.34, 130.81, 130.21, 129.79, 127.67, 127.41, 127.15, 126.99, 123.50, 122.90, 122.72, 120.29, 120.15, 119.03, 118.73, 117.13, 116.83, 116.61, 116.45, 116.24, 112.46, 112.35, 111.95, 111.68, 111.52, 110.06, 81.77, 81.06, 52.67, 52.24, 52.01, 48.59, 48.33, 29.85, 28.89, 28.52, 28.41.

**HPLC** (Sunfire C18, 60-95% of A in B, 5 min): t_{R} = 1.94 min.

**LC-MS (m/z):** 611.44 ([M+H]⁺).

### Example 22:

### Methyl 4-(((1RS,2S)-2-((tert-butoxycarbonyl)amino)-1-cyano-3-(1H-indol-3-yl)propyl)amino)-2',3',4',5'-tetrahydro-[1,1'-biphenyl]-3-carboxylate

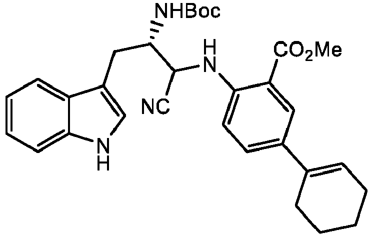

C₃₁H₃₆N₄O₄ 528.27·mol⁻¹

Obtained following Protocol A and Protocol D.

Purification by flash chromatography, using 0% to 60% of EtOAc in hexane gradient as eluants, furnished the desired compound as an epimeric mixture, ratio 1:1.5/*1R,2S:1S,2S* in 56 % yield
¹H-NMR (400 MHz, CDCl₃) δ (ppm): 8.26 (d, J = 8.4 Hz, 2H), 8.16 (s, 1.22H), 8.12 (s, 0.66H), 7.98 (d, J = 2.3 Hz, 1H), 7.95 (d, J = 2.3 Hz, 1H), 7.70 (d, J = 8.1 Hz, 1H), 7.58 (d, J = 7.9 Hz, 1H), 7.49 (dd, J = 8.7, 2.3 Hz, 1H), 7.41 - 7.36 (m, 3.66H), 7.25 - 7.19 (m, 2.74H), 7.15 (d, J = 8.1 Hz, 2.5H), 7.14 - 7.09 (m, 1.6H), 7.07 (d, *J* = 1.8 Hz, 0.66H), 6.79 (d, J = 8.4 Hz, 0.66H), 6.48 (d, J = 8.6 Hz, 1H), 6.04 (p, J = 2.3 Hz, 1H), 6.01 (s, 1H), 4.91 (d, J = 9.7 Hz, 1H), 4.80 (m, 1.66H), 4.64 (m, 1.66H), 4.50 - 4.39 (m, 2.66H), 3.88 (s, 2H), 3.85 (s, 3H), 3.40 - 3.26 (m, 2.4H), 3.19 (m, 2.4H), 2.39 - 2.31 (m, 4.7H), 2.22 - 2.15 (m, 4.7H), 1.81 - 1.72 (m, 5.5H), 1.69 - 1.61 (m, 5.5H), 1.50 (s, 9H), 1.41 (s, 6H).

¹³C-NMR (100 MHz, CDCl₃) δ (ppm): 169.09, 168.79, 155.51, 146.93, 146.54, 136.49, 135.31, 132.33, 132.24, 131.81, 131.41, 128.06, 128.01, 127.42, 127.26, 123.37, 123.29, 123.12, 122.73, 122.63, 120.18, 120.08, 118.97, 118.81, 118.02, 117.64, 112.24, 112.05, 111.98, 111.46, 111.40, 110.28, 110.17, 80.72, 52.63, 52.01, 51.87, 48.59, 48.47, 28.57, 28.46, 28.37, 27.40, 27.36, 25.94, 25.92, 23.15, 22.29.

**HPLC** (Sunfire C18, 80-95% of A in B, 5 min): t_{R} = 2.36 min.

**LC-MS (m/z):** 551.38 ([M+Na]⁺).

### Example 23:

### Methyl 2-(((1RS,2S)-2-((tert-butoxycarbonyl)amino)-1-cyano-3-(1H-indol-3-yl)propyl)amino)-5-(1-(tetrahydro-2H-pyran-2-yl)-1H-pyrazol-5-yl)benzoate

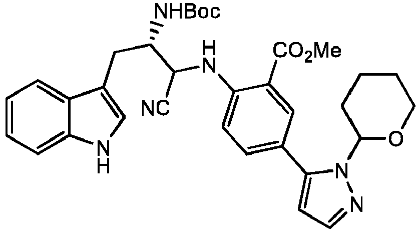

C₃₃H₃₈N₆O₅ 598.24·mol⁻¹

Obtained following Protocol A and Protocol D.

Purification by flash chromatography, using 20% to 50% of EtOAc in hexane gradient as eluants, furnished the desired compound as an epimeric mixture, ratio 1:1.5/*1R*,*2S*:*1S*,*2S* in 79 % yield
¹H-NMR (400 MHz, CDCl₃) δ (ppm): 8.53 (d, J = 7.8 Hz, 1H), 8.48 (dd, J = 8.1, 3.5 Hz, 0.66H), 8.25 (s, 1H), 8.22 (d, J = 2.2 Hz, 1H), 8.19 (d, J = 2.2 Hz, 0.66H), 8.16 (d, J = 2.3 Hz, 1H), 7.71 (d, J = 7.9 Hz, 1H), 7.59 (dt, J = 8.6, 2.6 Hz, 3.25H), 7.47 (d, J = 8.2 Hz, 1H), 7.42 - 7.37 (m, 2H), 7.24 - 7.21 (m, 1.32H), 7.20 - 7.17 (m, 1.66H), 7.15 (s, 0.66H), 7.14 - 7.11 (m, 1.32H), 6.93 (d, J = 7.6 Hz, 0.66H), 6.58 (d, J = 8.7 Hz, 1H), 6.30 (dd, J = 2.8, 1.8 Hz, 0.66H), 6.27 (dd, J = 4.3, 1.9 Hz, 1H), 5.14 (dd, J = 10.4, 2.6 Hz, 0.66H), 5.09 (dd, J = 10.4, 2.4 Hz, 1H), 4.95 (d, J = 9.8 Hz, 1H), 4.84 (m, 1H), 4.68 (m, 1H), 4.53 - 4.44 (m, 1.98H), 4.17 - 4.09 (m, 1.98H), 3.89 (s, 2H), 3.86 (s, 3H), 3.68 - 3.55 (m, 2.12H), 3.44 - 3.30 (m, 1.98H), 3.21 (dd, J = 14.7, 8.9 Hz, 1.98H), 2.66 - 2.52 (m, 2.25H), 2.06 (m, J = 8.8 Hz, 2.46H), 1.85 (t, J = 13.0 Hz, 2.64H), 1.76 (td, J = 8.0, 4.1 Hz, 1.87H), 1.56 (d, J = 10.3 Hz, 1H), 1.52 (s, 9H), 1.42 (s, 6H).

¹³C-NMR (100 MHz, CDCl₃) δ (ppm): 168.61, 168.31, 155.55, 148.31, 147.91, 143.45, 139.63, 139.60, 136.55, 135.59, 135.49, 135.22, 135.13, 132.74, 132.64, 123.18, 122.79, 122.69, 120.21, 120.09, 119.66, 119.55, 118.95, 118.73, 117.74, 117.32, 112.47, 112.42, 112.33, 112.22, 112.19, 111.54, 111.49, 110.13, 109.93, 106.24, 106.19, 106.17, 84.48, 84.45, 80.90, 67.85, 52.59, 52.18, 52.04, 48.42, 48.33, 29.80, 29.76, 28.47, 28.37, 25.04, 23.23, 20.37.

**HPLC** (Sunfire C18, 70-95% of A in B, 5 min): t_{R} = 1.57 min.

**LC-MS (m/z):** 621.40 ([M+Na]⁺).

### Example 24:

### Methyl 5-(benzo[d]oxazol-5-yl)-2-(((1RS,2S)-2-((tert-butoxycarbonyl)amino)-1-cyano-3-(1H-indol-3-yl)propyl)amino)benzoate

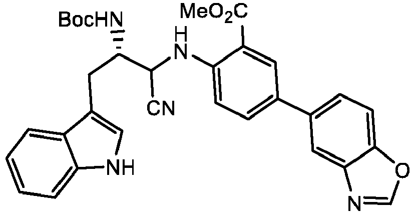

C₃₂H₃₁N₅O₅ 565.23·mol⁻¹

Obtained following Protocol A and Protocol D.

Purification by flash chromatography, using 10% to 40% of EtOAc in hexane gradient as eluants, furnished the desired compound as an epimeric mixture, ratio 1:1.5/*1R*,*2S*:*1S*,*2S* in 65 % yield
¹H-NMR (400 MHz, CDCl₃) δ (ppm): 8.41 (d, J = 8.3 Hz, 1H), 8.37 (s, 0.66H), 8.24 (d, J = 2.3 Hz, 0.66H), 8.21 (d, J = 2.3 Hz, 1.8H), 8.17 (s, 0.66H), 8.13 (s, 0.66H), 8.12 (s, 1H), 7.93 (d, J = 2.1 Hz, 0.66H), 7.90 (d, J = 2.1 Hz, 1H), 7.74 - 7.69 (m, 1.66H), 7.63 (d, J = 9.2 Hz, 1.27H), 7.61 - 7.55 (m, 3.66H), 7.54 (dd, J = 8.6, 1.8 Hz, 1.32H), 7.39 (t, J = 8.3 Hz, 2H), 7.23 (dq, J = 8.2, 1.2 Hz, 1.66H), 7.20 - 7.17 (m, 1.66H), 7.16 (s, 0.66H), 7.14 (dd, J = 2.8, 1.0 Hz, 0.66H), 7.12 (d, J = 1.1 Hz, 1H), 6.96 (d, J = 7.2 Hz, 0.66H), 6.61 (d, J = 9.0 Hz, 1H), 4.96 (d, J = 9.8 Hz, 1H), 4.84 (s, 1H), 4.68 (m, 1. 32H), 4.54 - 4.46 (m, 2H), 3.92 (s, 2H), 3.89 (s, 3H), 3.44 - 3.30 (m, 2H), 3.21 (dd, J = 14.9, 8.6 Hz, 2H), 1.52 (s, 9H), 1.42 (s, 6H).

¹³C-NMR (100 MHz, CDCl₃) δ (ppm): 168.83, 168.53, 155.55, 153.27, 153.24, 149.38, 149.34, 147.59, 147.20, 140.88, 140.84, 137.55, 136.53, 133.95, 133.57, 130.73, 130.69, 130.25, 130.17, 127.41, 127.21, 124.75, 123.16, 122.79, 122.68, 120.23, 120.11, 118.97, 118.78, 118.40, 118.38, 117.88, 117.49, 112.88, 112.75, 112.67, 111.51, 111.44, 111.19, 111.15, 110.23, 110.08, 80.82, 52.61, 52.17, 52.03, 48.57, 48.46, 28.69, 28.47, 28.39.

**HPLC** (Sunfire C18, 60-95% of A in B, 5 min): t_{R} = 3.35 min.

**LC-MS (m/z):** 566.23 ([M+H]⁺).

### Example 25:

### Methyl 2-(((1RS,2S)-2-((tert-butoxycarbonyl)amino)-1-cyano-3-(1H-indol-3-yl)propyl)amino)-5-(1-(2-morpholinoethyl)-1H-pyrazol-4-yl)benzoate

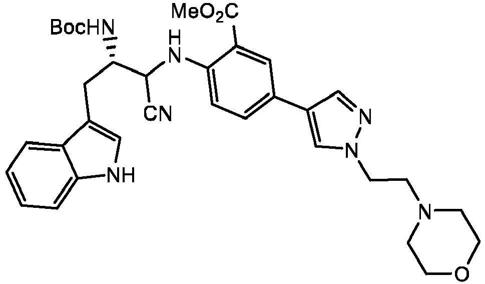

C₃₄H₄₁N₇O₅ 627.32·mol⁻¹

Obtained following Protocol A and Protocol D.

Purification by flash chromatography, using 2% to 5% of MeOH in DCM gradient as eluants, furnished the desired compound as an epimeric mixture, ratio 1:2/*1R*,*2S*:*1S*,*2S* in 23 % yield
¹H-NMR (400 MHz, CDCl₃) δ (ppm): 8.29 (t, J = 8.2 Hz, 1.5H), 8.20 (s, 1H), 8.16 (s, 0.5H), 8.06 (d, J = 2.3 Hz, 0.5H), 8.03 (d, J = 2.2 Hz, 1H), 7.73 - 7.65 (m, 5H), 7.58 (d, J = 7.1 Hz, 1H), 7.56 - 7.52 (m, 1H), 7.40 (q, J = 9.6 Hz, 4H), 7.21 (t, J = 8.2 Hz, 2H), 7.17 (s, 1H), 7.15 (s, 0.5H), 7.14 - 7.09 (m, 1.5H), 6.87 (s, 0.5H), 6.52 (d, J = 8.4 Hz, 1H), 4.93 (d, J = 9.4 Hz, 1H), 4.81 (s, 1H), 4.66 (s, 1.5H), 4.51 - 4.31 (m, 6H), 3.91 (s, 1.5H), 3.88 (s, 3H), 3.76 (s, 6H), 3.42 - 3.28 (m, 2.5H), 3.20 (dd, J = 15.0, 8.6 Hz, 2H), 2.96 (s, 3H), 2.58 (s, 6H), 1.50 (s, 9H), 1.41 (s, 5H).

¹³C-NMR (100 MHz, CDCl₃) δ (ppm): 168.77, 168.47, 155.53, 146.83, 146.44, 136.52, 132.08, 128.76, 127.23, 126.13, 123.14, 122.77, 122.66, 122.34, 120.21, 120.10, 118.97, 118.79, 112.87, 112.66, 111.49, 111.43, 110.26, 110.12, 80.79, 66.91, 58.28, 53.77, 52.57, 52.00, 48.52, 28.38, 25.01.

**HPLC** (Sunfire C18, 30-95% of A in B, 5 min): t_{R} = 2.97 min.

**LC-MS (m/z):** 628.47 ([M+H]⁺).

### Example 26:

### tert-Butyl ((1RS,2R)-1-((2-benzoyl-4-bromophenyl)amino)-1-cyano-3-(1H-indol-3-yl)propan-2-yl)carbamate

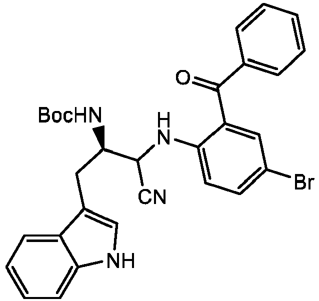

C₃₀H₂₉BrN₄O₃, 572.14 g.mol-1

Obtained following Protocol E using (2-amino-5-bromophenyl)(phenyl)methanone.

Purification by flash chromatography, using 20% to 30% of EtOAc in hexane gradient as eluants, furnished the desired compound as an epimeric mixture, ratio 1:2/*1S,2R:1R,2R* in 40 % yield
¹H-NMR (400 MHz, CDCl₃) δ (ppm): 8.76 (t, J = 8.7 Hz, 1.5H), 8.19 (s, 1H), 8.14 (s, 0.5H), 7.70 (d, J = 8.3 Hz, 1H), 7.64 - 7.52 (m, 8H), 7.52 - 7.44 (m, 4H), 7.40 (d, J = 8.2 Hz, 2H), 7.35 (d, J = 8.2 Hz, 0.5H), 7.23 (dd, J = 8.3, 1.3 Hz, 1.5H), 7.20 - 7.13 (m, 3H), 7.10 (d, J = 5.5 Hz, 1H), 6.87 (d, *J* = 8.1 Hz, 0.5H), 6.48 (d, J = 8.7 Hz, 1H), 4.92 (d, J = 9.2 Hz, 1H), 4.82 (d, J = 11.0 Hz, 1H), 4.63 (s, 1H), 4.46 (ddd, J = 7.8, 5.4, 2.5 Hz, 1.5H), 3.44 - 3.28 (m, 2H), 3.19 (dd, J = 14.8, 8.6 Hz, 2H), 1.45 (s, 9H), 1.40 (s, 4.5H).

¹³C-NMR (100 MHz, CDCl₃) δ (ppm): 198.44, 197.93, 155.53, 147.61, 147.20, 138.96, 137.86, 137.36, 137.22, 137.14, 136.54, 136.51, 132.05, 131.96, 129.52, 129.43, 128.54, 128.45, 127.31, 127.14, 123.17, 122.83, 122.70, 121.17, 120.25, 120.12, 118.94, 118.70, 117.46, 117.08, 114.31, 114.19, 111.54, 111.48, 110.14, 109.94, 108.55, 80.92, 52.47, 52.05, 48.43, 48.34, 28.50, 28.42, 28.36.

**HPLC** (Sunfire C18, 70-95% of A in B, 5 min): t_{R} = 3.02 min.

**LC-MS (m/z):** 595.21 ([M+Na]⁺).

### Example 27:

### Methyl 2-(((1RS,2R)-2-((tert-butoxycarbonyl)amino)-1-cyano-3-(1H-indol-3-yl)propyl)amino)-5-(1H-indol-5-yl)benzoate

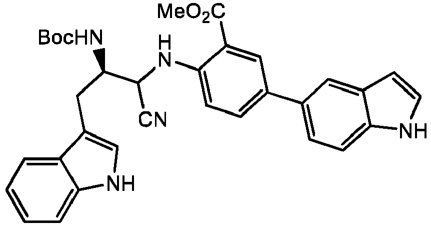

C₃₃H₃₃N₅O₄, 563.25 g·mol⁻¹

Obtained following Protocol A and Protocol D.

Purification by flash chromatography, using 20% to 50% of EtOAc in hexane gradient as eluants, furnished the desired compound as an epimeric mixture, ratio 1:2/*1S,2R:1R,2R* in 52 % yield
¹H-NMR (400 MHz, CDCl₃) δ (ppm): 8.33 (d, J = 8.4 Hz, 1.5H), 8.28 - 8.20 (m, 3.5H), 8.17 (s, 1H), 8.13 (s, 0.5H), 7.80 (s, 0.5H), 7.77 (s, 1H), 7.72 (d, J = 8.7 Hz, 1.5H), 7.62 (t, J = 8.4 Hz, 1.5H), 7.46 - 7.34 (m, 6H), 7.25 - 7.19 (m, 3.5H), 7.17 (d, J = 7.2 Hz, 2H), 7.13 (d, J = 7.2 Hz, 1H), 7.10 (d, J = 3.5 Hz, 0.5H), 6.91 (d, *J =* 7.2 Hz, 0.5H), 6.61 - 6.57 (m, 2.5H), 4.95 (d, J = 9.9 Hz, 1H), 4.84 (s, 1H), 4.67 (s, 1H), 4.55 - 4.45 (m, 1.5H), 3.92 (s, 1.5H), 3.89 (s, 3H), 3.36 (ddd, J = 26.0, 14.5, 5.0 Hz, 1.5H), 3.22 (dd, J = 14.7, 8.4 Hz, 1.5H), 1.52 (s, 9H), 1.42 (s, 4.5H).

¹³C-NMR (100 MHz, CDCl₃) δ (ppm): 169.09, 168.79, 155.52, 146.85, 146.45, 136.46, 135.14, 134.00, 133.63, 132.30, 132.05, 130.41, 130.34, 128.54, 128.51, 127.22, 124.96, 123.12, 122.71, 122.60, 121.41, 120.16, 120.06, 118.94, 118.79, 118.54, 118.04, 117.67, 112.76, 112.55, 112.46, 111.45, 111.39, 111.36, 110.22, 110.10, 102.99, 80.73, 52.62, 52.04, 51.90, 48.55, 48.44, 28.44, 28.35, 26.27.

**HPLC** (Sunfire C18, 60-95% of A in B, 5 min): t_{R} = 3.58 min.

**LC-MS (m/z):** 564.34 ([M+H]⁺).

### Example 28:

### Methyl 2-(((1RS,2R)-2-((tert-butoxycarbonyl)amino)-1-cyano-3-(1H-indol-3-yl)propyl)amino)-5-(2,3-dihydrobenzo[b][1,4]dioxin-6-yl)benzoate

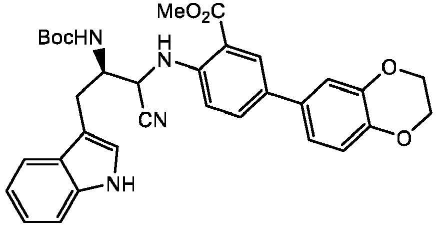

C₃₃H₃₄N₄O₆, 582.25 g·mol⁻¹

Obtained following Protocol A and Protocol D.

Purification by flash chromatography, using 20% to 40% of EtOAc in hexane gradient as eluants, furnished the desired compound as an epimeric mixture, ratio 1:2/*1S,2R:1R,2R* in 88 % yield
¹H-NMR (400 MHz, CDCl₃) δ (ppm): 8.33 (t, J = 8.7 Hz, 1.5H), 8.15 (d, J = 2.3 Hz, 1.5H), 8.12 (d, J = 2.4 Hz, 1.5H), 7.71 (d, J = 7.1 Hz, 1H), 7.62 (dd, J = 8.7, 2.3 Hz, 0.5H), 7.59 (d, J = 7.1 Hz, 0.5H), 7.53 - 7.49 (m, 1H), 7.39 (t, J = 7.9 Hz, 2H), 7.25 - 7.19 (m, 2H), 7.17 (d, J = 2.3 Hz, 1.5H), 7.15 (s, 0.5H), 7.12 (d, J = 8.1 Hz, 1H), 7.10 (d, J = 3.1 Hz, 0.5H), 7.07 (d, J = 2.2 Hz, 0.5H), 7.05 - 7.00 (m, 2H), 6.99 (d, J = 2.2 Hz, 0.5H), 6.90 (t, J = 8.5 Hz, 2.5H), 6.56 (d, J = 8.6 Hz, 1H), 4.93 (d, J = 9.8 Hz, 1H), 4.81 (s, 1H), 4.66 (s, 1.5H), 4.52 - 4.43 (m, 2H), 4.30 (s, 2H), 4.28 (s, 4H), 3.90 (s, 1.5H), 3.87 (s, 3H), 3.42 - 3.28 (m, 2H), 3.20 (dd, J = 14.6, 8.5 Hz, 2H) 1.50 (s, 9H), 1.41 (s, 4.5H).

¹³C-NMR (100 MHz, CDCl₃) δ (ppm): 168.94, 168.64, 155.52, 147.23, 146.84, 143.87, 143.83, 142.95, 142.91, 136.53, 133.79, 133.31, 132.93, 130.05, 129.96, 129.90, 127.42, 127.24, 123.13, 122.78, 122.67, 120.22, 120.12, 119.57, 118.99, 118.81, 117.95, 117.74, 117.69, 117.57, 115.24, 112.77, 112.58, 112.49, 111.48, 111.41, 110.28, 110.15, 80.78, 64.61, 52.62, 52.09, 51.96, 48.56, 48.44, 28.62, 28.47, 28.38.

**HPLC** (Sunfire C18, 70-95% of A in B, 5 min): t_{R} = 2.12 min.

**LC-MS (m/z):** 583.36 ([M+H]⁺).

### Example 29:

### Methyl 5-(6-acetamidopyridin-3-yl)-2-(((1RS,2S)-2-((tert-butoxycarbonyl)amino)-1-cyano-3-(1H-indol-3-yl)propyl)amino)benzoate

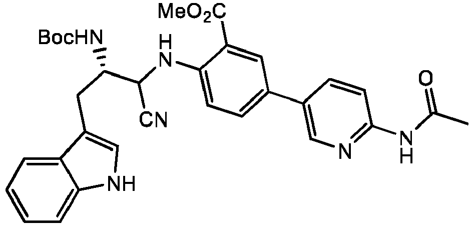

C₃₂H₃₄N₆O₅, 582.66 g·mol⁻¹

Obtained following Protocol B and Protocol D.

Purification by flash chromatography, using 0% to 40% of EtOAc in hexane gradient as eluants, furnished the desired compound as an epimeric mixture, ratio 1:1.5/*1R*,*2S*:*1S*,*2S* in 41 % yield.

¹H-NMR (400 MHz, CDCl₃) δ (ppm): 8.62 (s, 1H), 8.45 (d, J = 8.3 Hz, 1H), 8.41 (s, 0.66H), 8.39 (s, 1H), 8.36 (d, J = 2.4 Hz, 1H), 8.27 (dd, J = 15.2, 7.3 Hz, 3H), 8.20 (s, 0.66H), 8.14 (s, 0.66H), 8.13 (d, J = 2.3 Hz, 1H), 7.91 (s, 0.66H), 7.87 (dd, J = 8.7, 2.5 Hz, 1.33H), 7.73 - 7.64 (m, 2.66H), 7.60 - 7.51 (m, 2H), 7.51 - 7.44 (m, 2.66H), 7.42 - 7.36 (m, 2H), 7.24 - 7.20 (m, 1.66H), 7.20 - 7.18 (m, 1H), 7.16 (d, J = 7.7 Hz, 1H), 7.11 (q, J = 6.7 Hz, 2H), 6.98 (d, J = 11.5 Hz, 0.66H), 6.59 (d, J = 8.7 Hz, 1H), 4.97 (d, J = 9.7 Hz, 1H), 4.85 (s, 1H), 4.68 (s, 1H), 4.53 - 4.42 (m, 1.66H), 3.91 (s, 1.5H), 3.89 (s, 3H), 3.36 (ddd, J = 21.9, 15.0, 5.6 Hz, 2H), 3.24 - 3.17 (m, 2H), 2.25 (s, 2H), 2.24 (s, 3H), 1.51 (s, 9H), 1.42 (s, 6H).

¹³C-NMR (100 MHz, CDCl₃) δ (ppm): 168.94, 168.42, 168.15, 155.42, 149.75, 149.68, 147.84, 147.48, 143.74, 136.99, 136.42, 136.40, 132.99, 132.62, 132.17, 132.07, 132.00, 131.98, 129.92, 129.86, 128.60, 128.48, 127.26, 127.04, 123.04, 122.67, 122.56, 120.10, 119.98, 118.81, 118.60, 117.62, 117.21, 114.16, 112.89, 112.75, 112.52, 111.41, 111.35, 110.04, 109.85, 80.75, 52.40, 52.12, 51.99,48.38, 48.31, 28.63, 28.33, 28.25, 24.75.

**HPLC** (Sunfire C18, 50-95% of A in B, 5 min): t_{R} = 2.90 min.

**LC-MS (m/z):** 583 ([M+H]⁺).

### Example 30:

### Methyl 2-(((1RS,2S)-2-((tert-butoxycarbonyl)amino)-1-cyano-3-(1H-indol-3-yl)propyl)amino)-5-(1H-indazol-4-yl)benzoate

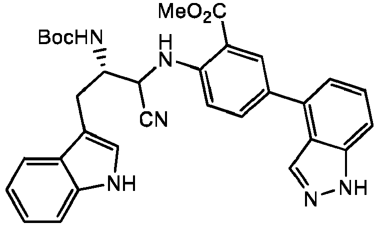

C₃₂H₃₂N₆O₄, 564.25 g·mol⁻¹

Obtained following Protocol A and Protocol D.

Purification by flash chromatography, using 10% to 40% of EtOAc in hexane gradient as eluants, furnished the desired compound as an epimeric mixture, ratio 1:1.3/*1R,2S:1S,2S* in 67 % yield
¹H-NMR (400 MHz, CDCl₃) δ (ppm): 8.48 (d, J = 8.3 Hz, 1H), 8.44 (d, J = 8.0 Hz, 0.66H), 8.33 (d, J = 2.3 Hz, 1H), 8.31 (d, J = 2.2 Hz, 1H), 8.23 (s, 2H), 8.18 (s, 1.66H), 7.79 - 7.65 (m, 4H), 7.60 (d, J = 7.9 Hz, 1H), 7.57 - 7.51 (m, 1H), 7.49 - 7.37 (m, 7H), 7.26 - 7.09 (m, 8H), 7.02 - 6.96 (m, 0.66H), 6.64 (d, J = 8.7 Hz, 1H), 5.00 (d, J = 9.7 Hz, 1H), 4.89 (d, J = 7.9 Hz, 1.32H), 4.70 (s, 1H), 4.52 (ddd, J = 13.5, 8.3, 4.9 Hz, 2H), 3.91 (s, 2H), 3.88 (s, 3H), 3.38 (ddd, J = 25.7, 14.8, 5.6 Hz, 2H), 3.23 (dd, J = 14.7, 8.6 Hz, 2H), 1.52 (s, 9H), 1.43 (s, 6H).

¹³C-NMR (100 MHz, CDCl₃) δ (ppm): 168.71, 168.42, 155.45, 147.74, 147.35, 140.63, 136.43, 134.81, 134.44, 134.26, 131.69, 128.91, 128.83, 127.30, 127.09, 123.06, 122.67, 122.55, 121.61, 120.10, 119.98, 119.84, 118.85, 118.66, 117.76, 117.38, 112.68, 112.51, 112.41, 111.40, 111.33, 110.09, 109.94, 108.28, 80.73, 52.54, 52.08, 51.95, 48.39, 48.27, 28.56, 28.35, 28.27.

**HPLC** (Sunfire C18, 60-95% of A in B, 5 min): t_{R} = 2.12 min.

**LC-MS (m/z):** 565 ([M+H]⁺).

### Example 31:

### tert-Butyl ((1RS,2S)-1-((2-(azepane-1-carbonyl)phenyl)amino)-1-cyano-3-(1H-indol-3-yl)propan-2-yl)carbamate

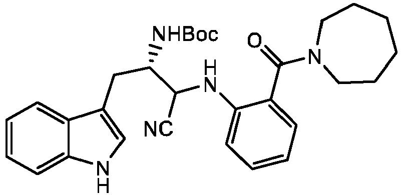

C₃₀H₃₇N₅O₃, 515.66 g·mol⁻¹

Obtained following Protocol E using (2-aminophenyl)(azepan-1-yl)methanone.

Purification by flash chromatography, using 0% to 40% of AcOEt in hexane gradient as eluants, furnished the desired compound as an epimeric mixture, ratio 1:3/*1R*,*2S*:*1S*,*2S* in 23% yield.

¹H-NMR (400 MHz, CDCl₃) δ (ppm): 8.25 (s, 1.33H), 7.67 (d, J = 7.9 Hz, 1H), 7.57 (d, J = 7.9 Hz, 0.33H), 7.37 (dd, J = 8.1, 3.2 Hz, 1.33H), 7.25 - 7.17 (m, 2.33H), 7.17 - 7.06 (m, 4H), 6.82 (dt, J = 11.5, 7.5 Hz, 2H), 6.59 (d, J = 8.2 Hz, 1H), 4.93 (d, J = 9.8 Hz, 1.33H), 4.59 (s, 0.33H), 4.46 (d, J = 5.8 Hz, 1H), 4.41 - 4.35 (m, 0.33H), 4.31 (d, J = 4.1 Hz, 1H), 3.68 - 3.54 (m, 3H), 3.34 (s, 6H), 3.24 (dd, J = 14.9, 6.1 Hz, 1.33H), 3.14 (dd, J = 14.6, 8.4 Hz, 1.66H), 1.89 - 1.75 (m, 4H), 1.58 - 1.52 (m, 5H), 1.46 (s, 9H), 1.43 - 1.40 (m, 2H).

¹³C-NMR (100 MHz, CDCl₃) δ (ppm): 170.42, 170.08, 155.80, 142.39, 136.38, 130.62, 130.28, 127.55, 127.16, 123.42, 123.12, 123.06, 119.99, 119.85, 118.69, 118.19, 117.60, 112.54, 111.39, 110.05, 109.86, 80.59, 53.15, 49.64, 49.43,46.13, 29.28, 28.30, 27.93, 27.31, 26.41.

**HPLC** (Sunfire C18, 60-95% of A in B, 5 min): t_{R} = 2.69 min.

**LC-MS (m/z):** 538 ([M+Na]⁺).

Diastereomeric ratios of Table 1 are expressed as the molar ratio of the diastereomer of configuration (R,S) to the diastereomer of configuration (S,S), whereby the diastereomer of configuration (R,S) is that wherein the carbon atom bearing the CN group is in the configuration (R) and the carbon atom bearing the NHBoc group is in the configuration (S); and whereby, similarly, the diastereomer of configuration (S,S) is that wherein the carbon atom bearing the CN group is in the configuration (S) and the carbon atom bearing the NHBoc group is in the configuration (S).

### Example 32: Anti-viral effect of compounds of formula (I)

### Measurement of protective effect against virus-induced cytopathic effect:

Vero-E6 cells were seeded onto 96-well plates (1×10⁴ cells/well). Candidate compounds were diluted from stock solutions in complete media [(DMEM) supplemented with 10 mM HEPES, 1X non-essential amino acids (Gibco), 100 U/mL penicillin-streptomycin (Gibco) and 2% fetal bovine serum (heat-inactivated at 56 °C for 30 min)] to achieve the desired concentration. A wide range of compound concentrations from 50 to 0.78 µM were tested in an initial experiment.

SARS-CoV-2 stock strain NL/2020 (provided by Dr. R. Molenkamp, Erasmus University Medical Center Rotterdam) was diluted to achieve a multiplicity of infection (MOI) of 0.001. Virus-compound mixture was used to inoculate the Vero-E6 monolayer. Cultures were maintained at 37 °C in a 5% CO₂ incubator for 72 h in a BSL3 facility. Cells were fixed using a 4% formaldehyde-PBS solution to finalize the experiment and inactivate the virus. Virus-induced cell death was revealed by staining with a 0.1% crystal violet solution in water-methanol for 30-60 minutes, time after which, plates were extensively washed with water and dried. Infected, as well as uninfected cells, were used as controls. Using crystal violet staining, the dilutions of compounds capable of protecting the cell monolayer from virus-induced cell death were determined. Concentration of the compound present at the lowest dilutions protecting 100% of the cell monolayer are averaged with the immediate lower dilution to estimate an effective concentration 50 (EC₅₀). The ratio between the highest and the lowest protective concentration (PCmax and PCmin) is used to estimate a preliminary therapeutic index, as it corresponds to the range of concentrations that are effective and non-cytotoxic to the cell monolayer.

### Biological evaluation of the antiviral activity in A549-ACE2 cells using viral antigen quantitation.

To determine the antiviral activity of the candidate compounds, SARS-CoV-2 stock strain NL/2020 (provided by Dr. R. Molenkamp, Erasmus University Medical Center Rotterdam) was diluted to achieve a multiplicity of infection (MOI) of 0.005 and mixed with non-toxic compound dilutions in complete culture media [(DMEM) supplemented with 10 mM HEPES, 1X non-essential amino acids (Gibco), 100 U/mL penicillin-streptomycin (Gibco) and 2% fetal bovine serum (heat-inactivated at 56 °C for 30 min)]. Virus-compound mixture was used to inoculate the A549-ACE2 cell monolayer (2 ×10⁴ cells/M96 well). Cultures were maintained at 37 °C in a 5% CO₂ incubator for 48 h in a BSL3 facility. Cells were subsequently fixed by 20 minutes incubation with a 4% formaldehyde-PBS solution. After extensive washes with PBS, infection efficiency was evaluated by the detection of intracellular SARS-CoV-2 N protein accumulation by immunofluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated conditions was used as reference (100%).

To evaluate the cytotoxicity profile of the selected compounds MTT assays were performed in A549-ACE2 cells. Uninfected cells were incubated with a range of compound for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated cells were used as reference (100%).

To determine the antiviral potency (EC₅₀ and EC₉₀) and cytotoxicity (CC₅₀) indexes of selected compounds A549-ACE2 cell line was inoculated at MOI 0.005 in the presence of increasing concentrations of the selected compounds, typically 0.4-50µM (unless lower doses were required) and incubated to allow infection propagation in the well. In this experimental setup, viral antigen staining in the cells is proportional to the overall virus propagation efficiency. Based on viral antigen staining, dose-response curves were built to estimate the concentrations of compound reducing SARS-CoV-2 infection efficiency by 50% (EC₅₀) or 90% (EC₉₀) as compared with the vehicle control. Parallel uninfected cultures were treated with the same compound concentrations and overall cell viability was estimated using an MTT assay to verify that antiviral doses are not overtly cytotoxic. Cytotoxicity index (CC₅₀) was estimated as the compound dose required to reduce cell viability by 50% as compared with the vehicle control. Using these procedures the EC₅₀, EC₉₀ and CC₅₀ values for the compounds of examples 1 to 31 was determined and is shown in table 1 below:

**TABLE 1**

| Example | EC₅₀ (µM) | EC₉₀ (µM) | CC₅₀ (µM) |
|---|---|---|---|
| 1 | 0.8 | 6 | >50 |
| 2 | 1.6 | 6 | >50 |
| 3 | 3.5 | 8 | >50 |
| 4 | 2 | 7,5 | >50 |
| 5 | 4 | 13 | >50 |
| 6 | 8 | 20 | >50 |
| 7 | 1.1 | 6 | >50 |
| 8 | 0.9 | 2 | >50 |
| 9 | <0.4 | 5 | 50 |
| 10 | 0.9 | 2 | >50 |
| 11 | 4 | 8 | 30 |
| 12 | 1.85 | 5 | >50 |
| 13 | 1.85 | 5 | 13 |
| 14 | 7 | 16 | >50 |
| 15 | 1 | 4 | 50 |
| 16 | 6 | 9 | 10 |
| 17 | 1 | 2 | >50 |
| 18 | 6 | 9 | 10 |
| 19 | 6 | 31 | >50 |
| 20 | 2 | 50 | >50 |
| 21 | 6 | >50 | >50 |
| 22 | 6 | 9 | 32 |
| 23 | 4 | 8 | 50 |
| 24 | 6 | 9 | >50 |
| 25 | 6 | 9 | >50 |
| 26 | 6 | 28 | 50 |
| 27 | 7 | 9 | 35 |
| 28 | 7 | 30 | >50 |
| 29 | 3 | 8 | >50 |
| 30 | 1 | 2 | 10 |
| 31 | 8 | 39 | 36 |

The results shown in Table 1 show the antiviral activity of the compounds against SARS-CoV-2 and their reduced cellular toxicity.

## Claims

1. A compound of formula (I) or a pharmaceutically acceptable salt thereof 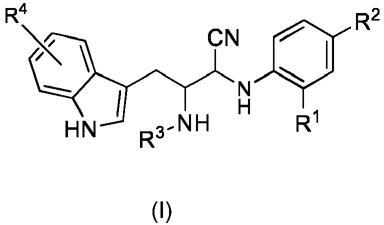
wherein R¹ is a group selected from the group consisting of hydrogen, halogen, carboxyl (-COOH), (C₁-C₄)alkyloxycarbonyl, (C₁-C₄)alkylcarbonyl, phenylcarbonyl and phenyl optionally substituted by a group selected from -CO-benzoimidazole and -CO-(5-7 saturated heterocycle comprising 1 or 2 heteroatoms selected from the group consisting of O, N and S);
R² is a group selected from the group consisting of:
a) hydrogen,
b) halogen,
c) a cycle selected from the group consisting of pyridinyl, pyrimidinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, 2,3-dihydrobenzo[b][1,4]dioxine and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group -G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom;
R³ is a group tert-butoxycarbonyl; and
R⁴ is a group selected from the group consisting of hydrogen, halogen, C₁₋₄-alkynylphenyl and 4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl
with the proviso that the compound is not one of the following compounds or their pharmaceutically acceptable salts:
methyl 4-(((2S)-2-((tert-butoxycarbonyl)amino)-1-cyano-3-(1H-indol-3-yl)propyl)-amino)-4'-morpholino-[1,1'-biphenyl]-3-carboxylate;
methyl 5-bromo-2-(((2S)-2-((tert-butoxycarbonyl)amino)-1-cyano-3-(1H-indol-3-yl)propyl)amino)benzoate;
(3S)-2-((4-Bromo-2-benzoylphenyl)amino)-4-(1H-indol-3-yl)-3-(tert-butoxycarbonylamino) butanenitrile;
(3S)-3-(tert-Butoxycarbonylamino)-4-(1H-indol-3-yl)-2-((2-benzoyl-4-chlorophenyl)amino) butanenitrile;
methyl 5-bromo-2-(((2S)-2-((tert-butoxycarbonyl)amino)-1-cyano-3-(1H-indol-3-yl)propyl)amino)benzoate;
methyl 2-(((2S)-2-((tert-butoxycarbonyl)amino)-1-cyano-3-(1H-indol-3-yl)propyl)amino)-5-(2,3-dihydrobenzo[b][1,4]dioxin-6-yl)benzoate;
methyl 4-(((2S)-2-((tert-butoxycarbonyl)amino)-1-cyano-3-(1H-indol-3-yl)propyl)amino)-3'-morpholino-[1,1'-biphenyl]-3-carboxylate;
ethyl 2-(((2S)-2-((tert-butoxycarbonyl)amino)-1-cyano-3-(1H-indol-3-yl)propyl)amino)-benzoate;
methyl 4-bromo-2-(((2S)-2-((tert-butoxycarbonyl)amino)-1-cyano-3-(1H-indol-3-yl)propyl)-amino)benzoate;
methyl 4-(((2S)-2-((tert-butoxycarbonyl)amino)-1-cyano-3-(1H-indol-3-yl)propyl)amino)-4'-(tert-butyl)-[1,1'-biphenyl]-3-carboxylate;
methyl 2-bromo-6-(((2S)-2-((tert-butoxycarbonyl)amino)-1-cyano-3-(1H-indol-3-yl)propyl)-amino)benzoate;
methyl 4-(((2S)-2-((tert-butoxycarbonyl)amino)-1-cyano-3-(1H-indol-3-yl)propyl)amino)-4'-ethyl-[1,1'-biphenyl]-3-carboxylate;
methyl 4-(((2S)-2-((tert-butoxycarbonyl)amino)-1-cyano-3-(1H-indol-3-yl)propyl)amino)-4'-cyclopropyl-[1,1'-biphenyl]-3-carboxylate;
methyl 4-(((2S)-2-((tert-butoxycarbonyl)amino)-1-cyano-3-(1H-indol-3-yl)propyl)amino)-4'-(4-methylpiperazin-1-yl)-[1,1'-biphenyl]-3-carboxylate;
methyl 4-(((2S)-2-((tert-butoxycarbonyl)amino)-1-cyano-3-(1H-indol-3-yl)propyl)amino)-2'-fluoro-5'-isopropoxy-[1,1'-biphenyl]-3-carboxylate;
methyl 2-(((2S)-2-((tert-butoxycarbonyl)amino)-1-cyano-3-(1H-indol-3-yl)propyl)amino)-5-(6-(pyrrolidin-1-yl)pyridin-3-yl)benzoate;
methyl 2-(((2S)-2-((tert-butoxycarbonyl)amino)-1-cyano-3-(1H-indol-3-yl)propyl)amino)-5-(6-methylpyridin-3-yl)benzoate;
methyl 2-(((2S)-2-((tert-butoxycarbonyl)amino)-1-cyano-3-(1H-indol-3-yl)propyl)amino)-5-(6-morpholinopyridin-3-yl)benzoate;
methyl 2-(((2S)-2-((tert-butoxycarbonyl)amino)-1-cyano-3-(1H-indol-3-yl)propyl)amino)-5-(6-methoxypyridin-3-yl)benzoate;
methyl 2-(((2S)-2-((tert-butoxycarbonyl)amino)-1-cyano-3-(1H-indol-3-yl)propyl)amino) benzoate;
tert-Butyl 2-(((2S)-2-((tert-butoxycarbonyl)amino)-1-cyano-3-(1H-indol-3-yl)propyl)amino)-benzoate;
methyl 4-(((2S)-2-((tert-butoxycarbonyl)amino)-1-cyano-3-(1H-indol-3-yl)propyl)amino)-4'-butyl-[1,1'-biphenyl]-3-carboxylate;
methyl 4-(((2S)-2-((tert-butoxycarbonyl)amino)-1-cyano-3-(1H-indol-3-yl)propyl)amino)-4'-(diethylamino)-[1,1'-biphenyl]-3-carboxylate;
methyl 4-(((2S)-2-((tert-butoxycarbonyl)amino)-1-cyano-3-(1H-indol-3-yl)propyl)amino)-4'-(cyclopropyl)-[1,1'-biphenyl]-3-carboxylate;
methyl 4-(((2S)-2-((tert-butoxycarbonyl)amino)-1-cyano-3-(1H-indol-3-yl)propyl)amino)-4'-propoxy-[1,1'-biphenyl]-3-carboxylate;
methyl 4-(((2S)-2-((tert-butoxycarbonyl)amino)-1-cyano-3-(1H-indol-3-yl)propyl)amino)-3'-(tert-butyl)-[1,1'-biphenyl]-3-carboxylate;
methyl 4-(((2S)-2-((tert-butoxycarbonyl)amino)-1-cyano-3-(1H-indol-3-yl)propyl)amino)-3'-ethyl-[1,1'-biphenyl]-3-carboxylate;
methyl 4-(((2S)-2-((tert-butoxycarbonyl)amino)-1-cyano-3-(1H-indol-3-yl)propyl)amino)-3'-(pyrrolidin-1-yl)-[1,1'-biphenyl]-3-carboxylate;
methyl 4-(((2S)-2-((tert-butoxycarbonyl)amino)-1-cyano-3-(1H-indol-3-yl)propyl)amino)-3'-(dimethylamino)-[1,1'-biphenyl]-3-carboxylate;
methyl 4-(((2S)-2-((tert-butoxycarbonyl)amino)-1-cyano-3-(1H-indol-3-yl)propyl)amino)-3'-(trifluoromethoxy)-[1,1'-biphenyl]-3-carboxylate;
methyl 2-(((2S)-2-((tert-butoxycarbonyl)amino)-1-cyano-3-(1H-indol-3-yl)propyl)amino)-5-(6-(4-isopropylpiperazin-1-yl)pyridin-3-yl)benzoate;
methyl 2-(((2S)-2-((tert-butoxycarbonyl)amino)-1-cyano-3-(1H-indol-3-yl)propyl)amino)-5-(2-ethoxypyrimidin-5-yl)benzoate;
methyl 4-(((2S)-2-((tert-butoxycarbonyl)amino)-1-cyano-3-(1H-indol-3-yl)propyl)amino)-4'-butyl-[1,1'-biphenyl]-3-carboxylate;
methyl 3'-benzyl-4-(((2S)-2-((tert-butoxycarbonyl)amino)-1-cyano-3-(1H-indol-3-yl)propyl)-amino)-[1,1'-biphenyl]-3-carboxylate;
2-(((2S)-2-(tert-butoxycarbonylamino)-1-cyano-3-(1H-indol-3-yl)propyl)amino)-N-benzyl-benzamide;
(3S)-2-((2-bromophenyl)amino)-4-(1H-indol-3-yl)-3-(tert-butoxycarbonylamino) butanenitrile;
methyl 2-(((2R)-2-((tert-butoxycarbonyl)amino)-1-cyano-3-(1H-indol-3-yl)propyl)amino)-benzoate;
(3S)-3-(tert-butoxycarbonylamino)-2-((2-acetylphenyl)amino)-4-(1H-indol-3-yl)butanenitrile;
methyl 4-(((2S)-2-((tert-butoxycarbonyl)amino)-1-cyano-3-(1H-indol-3-yl)propyl)amino)-benzoate;
tert-butyl ((2S)-1-((2-benzoylphenyl)amino)-1-cyano-3-(1H-indol-3-yl)propan-2-yl)carbamate; and
methyl 2-(((2S)-2-((tert-butoxycarbonyl)amino)-1-cyano-3-(1H-indol-3-yl)propyl)amino)benzoate.

2. A compound of formula (I) as defined in claim 1 that is a compound of formula (I') or a pharmaceutically acceptable salt thereof

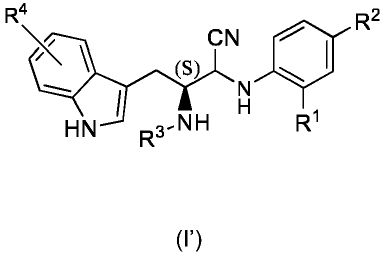

3. A compound of formula (I) as defined in any one of claims 1 to 2 or a pharmaceutically acceptable salt thereof wherein R¹ is methyloxycarbonyl.

4. A compound of formula (I) as defined in any one of claims 1 to 3 or a pharmaceutically acceptable salt thereof wherein R² is a cycle selected from the group consisting of pyridinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group -G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom;

5. A compound of formula (I) as defined in any one of claims 1 to 4 or a pharmaceutically acceptable salt thereof wherein R⁴ is a group selected from the group consisting of hydrogen, bromine, 4-ethynylphenyl and 4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl.

6. A compound of formula (I) as defined in any one of claims 1 to 7 or a pharmaceutically acceptable salt thereof that is selected from the group consisting of:
• Methyl 4-(((1RS,2S)-3-(5-bromo-1H-indol-3-yl)-2-((tert-butoxycarbonyl)amino)-1-cyanopropyl)amino)-4'-morpholino-[1,1'-biphenyl]-3-carboxylate
• Methyl 4-(((1RS,2S)-3-(6-bromo-1H-indol-3-yl)-2-((tert-butoxycarbonyl)amino)-1-cyanopropyl)amino)-4'-morpholino-[1,1'-biphenyl]-3-carboxylate
• Methyl 4-(((1RS,2S)-3-(7-bromo-1H-indol-3-yl)-2-((tert-butoxycarbonyl)amino)-1-cyanopropyl)amino)-4'-morpholino-[1,1'-biphenyl]-3-carboxylate
• Methyl 4-(((1RS,2S)-3-(4-bromo-1H-indol-3-yl)-2-((tert-butoxycarbonyl)amino)-1-cyanopropyl)amino)-4'-morpholino-[1,1'-biphenyl]-3-carboxylate
• Methyl 4-(((1RS,2S)-2-((tert-butoxycarbonyl)amino)-1-cyano-3-(5-phenyl-1H-indol-3-yl)propyl)amino)-4'-morpholino-[1,1'-biphenyl]-3-carboxylate
• Methyl 4-(((1RS,2S)-2-((tert-butoxycarbonyl)amino)-1-cyano-3-(4-(4-ethynylphenyl)-1H-indol-3-yl)propyl)amino)-4'-morpholino-[1,1'-biphenyl]-3-carboxylate
• Methyl 2-(((1RS,2S)-2-((tert-butoxycarbonyl)amino)-1-cyano-3-(1H-indol-3-yl)propyl)amino)-5-(2-morpholinopyridin-4-yl)benzoate
• Methyl 4-(((1RS,2S)-2-((tert-butoxycarbonyl)amino)-1-cyano-3-(1H-indol-3-yl)propyl)amino)-4'-(morpholine-4-carbonyl)-[1,1'-biphenyl]-3-carboxylate
• Methyl 2-(((1RS,2S)-2-((tert-butoxycarbonyl)amino)-1-cyano-3-(1H-indol-3-yl)propyl)amino)-5-(1H-indol-5-yl)benzoate
• Methyl 4-(((1RS,2S)-2-((tert-butoxycarbonyl)amino)-1-cyano-3-(1H-indol-3-yl)propyl)amino)-4'-(morpholinomethyl)-[1,1'-biphenyl]-3-carboxylate
• Methyl 2-(((1RS,2S)-2-((tert-butoxycarbonyl)amino)-1-cyano-3-(1H-indol-3-yl)propyl)amino)-5-(1H-indol-6-yl)benzoate
• Methyl 5-(benzofuran-2-yl)-2-(((1RS,2S)-2-((tert-butoxycarbonyl)amino)-1-cyano-3-(1H-indol-3-yl)propyl)amino)benzoate
• Methyl 5-(benzo[b]thiophen-2-yl)-2-(((1RS,2S)-2-((tert-butoxycarbonyl)amino)-1-cyano-3-(1H-indol-3-yl)propyl)amino)benzoate
• tert-Butyl 4-(4-(((1RS,2S)-2-((tert-butoxycarbonyl)amino)-1-cyano-3-(1H-indol-3-yl)propyl)amino)-3-(methoxycarbonyl)phenyl)-3,6-dihydropyridine-1(2H)-carboxylate
• Methyl 5-(1H-benzo[d]imidazol-5-yl)-2-(((1RS,2S)-2-((tert-butoxycarbonyl)amino)-1-cyano-3-(1H-indol-3-yl)propyl)amino)benzoate
• Methyl 2-(((1RS,2S)-2-((tert-butoxycarbonyl)amino)-1-cyano-3-(1H-indol-3-yl)propyl)amino)-5-(1H-indazol-5-yl)benzoate
• Methyl 5-(1-benzyl-1H-pyrazol-4-yl)-2-(((1RS,2S)-2-((tert-butoxycarbonyl)amino)-1-cyano-3-(1H-indol-3-yl)propyl)amino)benzoate
• Methyl 2-(((1RS,2S)-2-((tert-butoxycarbonyl)amino)-1-cyano-3-(1H-indol-3-yl)propyl)amino)-5-(1H-pyrrolo[2,3-b]pyridin-3-yl)benzoate
• tert-Butyl 5-(4-(((1RS,2S)-2-((tert-butoxycarbonyl)amino)-1-cyano-3-(1H-indol-3-yl)propyl)amino)-3-(methoxycarbonyl)phenyl)-1H-indole-1-carboxylate
• Methyl 4-(((1RS,2S)-2-((tert-butoxycarbonyl)amino)-1-cyano-3-(4-(4-(1-(14-oxo-17-((3aR,4R,6aS)-2-oxohexahydro-1H-thieno[3,4-d]imidazol-4-yl)-4,7,10-trioxa-13-azaheptadecyl)-1H-1,2,3-triazol-4-yl)phenyl)-1H-indol-3-yl)propyl)amino)-4'-morpholino-[1,1'-biphenyl]-3-carboxylate
• Methyl 4-(((1RS,2S)-2-((tert-butoxycarbonyl)amino)-1-cyano-3-(1H-indol-3-yl)propyl)amino)-4'-fluoro-3'-(2H-tetrazol-5-yl)-[1,1'-biphenyl]-3-carboxylate
• Methyl 4-(((1RS,2S)-2-((tert-butoxycarbonyl)amino)-1-cyano-3-(1H-indol-3-yl)propyl)amino)-2',3',4',5'-tetrahydro-[1,1'-biphenyl]-3-carboxylate
• Methyl 2-(((1RS,2S)-2-((tert-butoxycarbonyl)amino)-1-cyano-3-(1H-indol-3-yl)propyl)amino)-5-(1-(tetrahydro-2H-pyran-2-yl)-1H-pyrazol-5-yl)benzoate
• Methyl 5-(benzo[d]oxazol-5-yl)-2-(((1RS,2S)-2-((tert-butoxycarbonyl)amino)-1-cyano-3-(1H-indol-3-yl)propyl)amino)benzoate
• Methyl 2-(((1RS,2S)-2-((tert-butoxycarbonyl)amino)-1-cyano-3-(1H-indol-3-yl)propyl)amino)-5-(1-(2-morpholinoethyl)-1H-pyrazol-4-yl)benzoate
• tert-Butyl ((1RS,2R)-1-((2-benzoyl-4-bromophenyl)amino)-1-cyano-3-(1H-indol-3-yl)propan-2-yl)carbamate
• Methyl 2-(((1RS,2R)-2-((tert-butoxycarbonyl)amino)-1-cyano-3-(1H-indol-3-yl)propyl)amino)-5-(1H-indol-5-yl)benzoate
• Methyl 2-(((1RS,2R)-2-((tert-butoxycarbonyl)amino)-1-cyano-3-(1H-indol-3-yl)propyl)amino)-5-(2,3-dihydrobenzo[b][1,4]dioxin-6-yl)benzoate
• Methyl 5-(6-acetamidopyridin-3-yl)-2-(((1RS,2S)-2-((tert-butoxycarbonyl)amino)-1-cyano-3-(1 H-indol-3-yl)propyl)amino)benzoate
• Methyl 2-(((1RS,2S)-2-((tert-butoxycarbonyl)amino)-1-cyano-3-(1H-indol-3-yl)propyl)amino)-5-(1H-indazol-4-yl)benzoate
• tert-Butyl ((1RS,2S)-1-((2-(azepane-1-carbonyl)phenyl)amino)-1-cyano-3-(1H-indol-3-yl)propan-2-yl)carbamate

7. A composition comprising a compound as defined in any of claims 1 to 6 or a pharmaceutically acceptable salt thereof and one or more pharmaceutically acceptable diluent or carrier.

8. A compound as defined in any of claims 1 to 6 or a pharmaceutically acceptable salt thereof, or a composition as defined in claim 7 for use in medicine.

9. A compound as defined in any of claims 1 to 6 or a pharmaceutically acceptable salt thereof, or a composition as defined in claim 7 for use in the treatment or prevention of viral infection by coronavirus.

10. Process for the preparation of a compound of formula (I) as defined in any of claims 1 to 6 comprising the steps of:
(a) contacting a compound of formula (II) with a compound of formula (III) 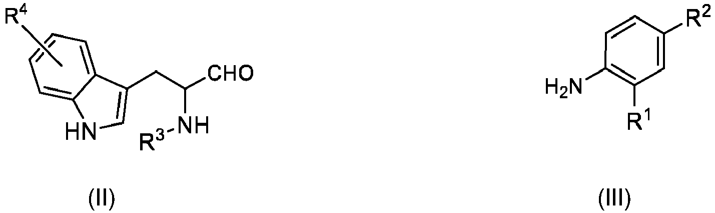 in order to form a compound of formula (IV) 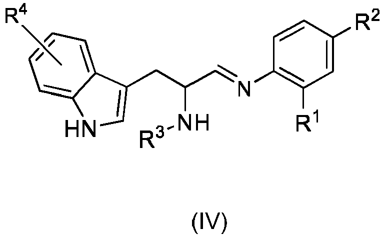 wherein R¹, R², R³ and are as defined in any one of claims 1 to 6,
and (b) treating the product of step (a) with a cyanide source in order to form a product of formula (I) as defined in claim 1.

11. Process for the preparation of a compound of formula (I) as defined in claim 10 comprising the steps of:
(a) contacting a compound of formula (II) with a compound of formula (Illa) 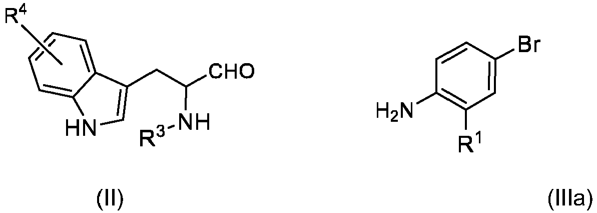 in order to form a compound of formula (IVa) 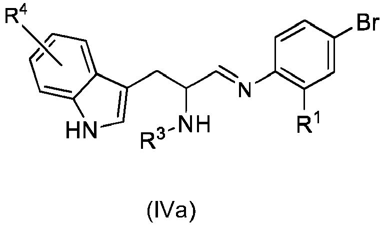 wherein R¹, R³ and R⁴ are as defined in any one of claims 1 to 6,
(b) treating the product of step (a) with a cyanide source in order to form a product of formula (V) 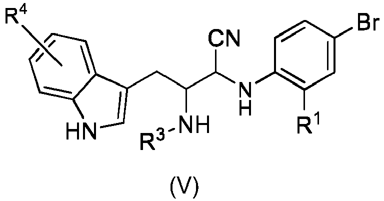 and (c) the step of contacting the compound of formula (V) with a compound of formula R³-B(OH)₂ or with a compound of formula 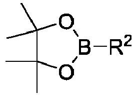 in the presence of a catalytically effective amount of a cross-coupling catalyst, wherein R³ is a cycle selected from the group consisting of pyridinyl, pyrimidinyl, indolyl, indazolyl, pyrazolyl, tetrahydropyridinyl, cyclohexenyl, benzoimidazolyl, pyrrolo[2,3-b]pyridinyl, benzofuranyl, benzoxazolyl, benzo[b]thiophenyl, and phenyl, said cycle being optionally substituted at any available position with one or two groups selected from the group consisting of (C₁-C₄)alkyloxycarbonyl(CH₂)ₘ, halogen, dioxolane, (C₁-C₄)alkyl, (C₁-C₄)alkyloxy, trilfuoromethyloxy, di((C₁-C₄)alkyl)amino, acetamido, benzyl and a group - G¹-(5 to 6-membered saturated or unsaturated heterocyclic ring), wherein G¹ is selected from direct bond, -CO-, -CH₂- and -CH₂-CH₂- and the members of the heterocyclic ring are selected from the group consisting of C, CH, CH₂, O, N and NH, said heterocyclic ring being optionally substituted at any available position with a (C₁-C₄)alkyl or an halogen atom.

12. Process for the preparation of a compound of formula (I) as defined in any one of claims 1 to 6 wherein R⁴ is a C₁₋₄-alkynylphenyl group, comprising the step of contacting the compound of formula (Ib) 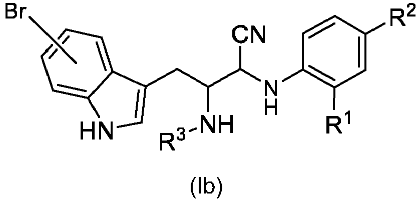 wherein R¹, R² and R³ are as defined for the compounds of formula (I) with a C₁₋₄-alkynylphenylboronic acid in the presence of a catalytically effective amount of a cross-coupling catalyst.
